(19) Europäisches Patentamt
European Patent Office
Office européen des brevets

(11) Publication number: **0 488 317 B1**

# (12) EUROPEAN PATENT SPECIFICATION

(45) Date of publication of patent specification: **14.06.95**

(51) Int. Cl.6: **C12N 15/12**, C12P 21/02, C07K 14/00, A61K 38/00

(21) Application number: **91120438.6**

(22) Date of filing: **28.11.91**

(54) **Thrombin-binding substance and process for preparing the same.**

(30) Priority: **30.11.90 JP 335720/90**
**25.02.91 JP 30271/91**

(43) Date of publication of application:
**03.06.92 Bulletin 92/23**

(45) Publication of the grant of the patent:
**14.06.95 Bulletin 95/24**

(84) Designated Contracting States:
**AT BE CH DE DK ES FR GB GR IT LI LU NL SE**

(56) References cited:

**BIOCHEMICAL AND BIOPHYSICAL RESEARCH COMMUNICATIONS vol. 171, no. 2, 14 September 1990, DULUTH, MINNESOTA US pages 729 - 737 K. NAWA ET AL. 'PRESENCE AND FUNCTION OF CHONDROITIN-4-SULFATE ON RECOMBINANT HUMAN SOLUBLETHROMBOMODULIN'**

(73) Proprietor: **KOWA COMPANY, LTD.**
**6-29, 3-chome**
**Nishiki**
**Naka-ku**
**Nagoya**
**Aichi-ken (JP)**

(72) Inventor: **Doi, Takeshi Paru Haitsu**
**1-17-1, Kannondai**
**Tsukuba-shi,**
**Ibaraki (JP)**
Inventor: **Iwasaki, Akio**
**1-14-1-302, Tokiwadaira**
**Matsudo-shi,**
**Chiba (JP)**
Inventor: **Saino, Yushi**
**3-19-2-304, Kamishakujii**
**Nerima-ku,**
**Tokyo (JP)**
Inventor: **Kimura, Shigeru**
**3-59-10, Nangai**
**Higashiyamato-shi,**
**Tokyo (JP)**
Inventor: **Ohkuchi, Masao**
**3-9-5, Nakaarai**
**Tokorozawa-shi,**
**Saitama (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

JOURNAL OF BIOLOGICAL CHEMISTRY. (MICROFILMS) vol. 265, no. 9, 25 March 1990, BALTIMORE, MD US pages 4915 - 4922 K.T. PREISSNER ET AL. 'Domain Structure of the Endothelial Cell Receptor Thrombomodulin as Deduced from Modulation of Its Anticoagulant Functions'

JOURNAL OF BIOLOGICAL CHEMISTRY. (MICROFILMS) vol. 264, no. 9, 25 March 1989, BALTIMORE, MD US pages 4872 - 4876 K. SUZUKI ET AL. 'A Domain Composed of Epidermal Growth Factor-like Structures of Human Thrombomodulin Is Essentialfor Thrombin Binding and for Protein C Activation'

(74) Representative: **Wächtershäuser, Günter, Prof. Dr.**
**Patentanwalt,**
**Tal 29**
**D-80331 München (DE)**

## Description

### BACKGROUND OF THE INVENTION

Field of the Invention:

The present invention relates to a novel thrombin-binding substance, a DNA fragment encoding the amino acid sequence of said thrombin-binding substance, a recombinant vector comprising said DNA fragment, a transformed cell harboring said recombinant vector, an anticoagulant composition comprising said thrombin-binding substance which has platelet aggregation inhibitory activity, and a process for the preparation of said thrombin-binding substance.

Description of the Background Art:

A great deal of work have been done regarding the role that thrombin plays as a proteolytic enzyme in the blood coagulation control mechanism and the mechanism of blood coagulation has been elucidated for the most part.

A publication reports that thrombin activates Protein C which is said to act on the fibrinolytic and anticoagulant systems and that there is a certain substance in extracts of rabbit lung tissues which functions as a coenzyme for the activation mechanism. Such a substance was named thrombomodulin [N. L. Esmon et al, *J. Biological Chemistry*, *257*, (2), 859-864 (1982)].

N. Aoki, et al reported that a human thrombomodulin separated from human placenta with a molecular weight of about 71,000 under nonreducing conditions had characteristics similar to the thrombomodulin reported by Esmon et al [*Thromb. Res.*, *37*, 353-364 (1985)].

I. Maruyama et al compared the activities of human thrombomodulin separated from human placenta having a molecular weight of about 75,000 with the activities of the above-mentioned rabbit thrombomodulin. They reported that the two thrombomodulins were equivalent in activity [*J. Clin. Invest.*, *75*, 987-991 (1985)].

H. Ishii et al reported that human plasma and human urine contained substances having the same activities as thrombomodulin and that the molecular weights of such substances in plasma were 63,000 and 54,000 [*J. Clin. Invest.*, *76*, 2178-2181 (1985)].

The present inventors previously discovered two types of thrombin-binding substances in human urine. They are different from the above-mentioned substances; having smaller molecular weights, i.e., about 39,000 and 31,000 under nonreducing conditions. The present inventors filed a patent application on these substances (Japanese Patent Laid-open (kokai) No. 146898/1988).

Furthermore, the present inventors separated two types of thrombin-binding substances (A) and (B) from human urine and a culture broth of cells derived from human tissues, and established a process for producing large amounts of these thrombin-binding substances in a stable manner. The present inventors previously filed patent applications on the thrombin-binding substances and the process (European Patent Publication No. 455,681).

The present inventors obtained a human urine derived thrombin-binding substance using a recombinant DNA technique (r-UTM) and filed a patent application on this process (Japanese Patent Application No. 54446/1991).

Thrombomodulin from rabbit lungs is known to increase the activity of antithrombin III [K. T. Preissner et al, *J. Biological Chemistry*, *265*, 4915-4922 (1990)]. Such an activity, however, is not possessed by thrombomodulin from bovine [H. V. Jakubowski et al, *J. Biological Chemistry*, *261*, 3876 (1986)], and thrombomodulin from human placenta inhibits the activity of antithrombin III [K. Hirahara et al, *Thrombo. Res.*, *57*, 117-126 (1990)].

Also, two soluble thrombomodulins produced by genetic manipulation techniques are known in the art. One is known to increase the activity of antithrombin III and another is known to possess no such capability [K. Nawa et al, *Biochem. Biophys. Res.*, *171*, 729-737 (1990)]. These thrombomodulins, however, are known to inhibit the thrombin coagulation in platelet which plays an important role in the blood coagulation system, but not to have an ADP coagulation effect [N. L. Esmon, *J. Biological Chemistry*, *258*, 12238-12242 (1983)].

Promoting the antithrombin III activity and the platelet aggregation inhibitory activity in human thrombomodulins and other thrombin-binding substances has therefore been desired.

SUMMARY OF THE INVENTION

In view of this situation, the present inventors have undertaken extensive studies and found that a transformant prepared by transforming a host cell with a recombinant vector into which a DNA fragment obtained by combining a specific DNA fragment at the 3'-end of a DNA fragment encoding a thrombin-binding substance derived from human urine is combined can produce a thrombin-binding substance derived from human urine capable of increasing an antithrombin III activity and inhibiting platelet aggregation.

Accordingly, an object of the present invention is to provide a novel thrombin-binding substance having the following amino acid sequence (hereinafter referred to as "Sequence A"), a DNA fragment having the nucleotide sequence encoding Sequence A, a recombinant vector comprising said DNA fragment and a replicable vector, and a transformed cell harboring said recombinant vector.

<Sequence A>

AlaProAlaGluProGlnProGlyGlySerGlnCysValGluHisAspCysPheAlaLeu

TyrProGlyProAlaThrPheLeuAsnAlaSerGlnIleCysAspGlyLeuArgGlyHis

LeuMetThrValArgSerSerValAlaAlaAspValIleSerLeuLeuLeuAsnGlyAsp

GlyGlyValGlyArgArgArgLeuTrpIleGlyLeuGlnLeuProProGlyCysGlyAsp

ProLysArgLeuGlyProLeuArgGlyPheGlnTrpValThrGlyAspAsnAsnThrSer

TyrSerArgTrpAlaArgLeuAspLeuAsnGlyAlaProLeuCysGlyProLeuCysVal

AlaValSerAlaAlaGluAlaThrValProSerGluProIleTrpGluGluGlnGlnCys

GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids and Y1 and Y2 represent any arbitrary amino acids.

Another object of the present invention is to provide an anticoagulant composition comprising said thrombin-binding substance and exhibiting platelet aggregation inhibitory activity.

Still another object of the present invention is to provide a process for the preparation of said thrombin-binding substance.

Other objects, features and advantages of the invention will hereinafter become more readily apparent from the following description.

## BRIEF DESCRIPTION OF THE DRAWING

Figure 1 is a scheme illustrating the structure of expression vector, pCDM-GAG-UTM1 and pCDM-GAG-UTM2, of the present invention.

## DETAILED DESCRIPTION OF THE INVENTION AND PREFERRED EMBODIMENTS

The thrombin-binding substance of the present invention can be prepared, for example, according to the following process. A template DNA is first prepared by cutting a human placenta genome DNA with a suitable restriction endonuclease. The template DNA is screened using, as a probe, a DNA primer synthesized referring to a nucleotide sequence of a known human thrombomodulin gene [Shirai, T et al, *J. Biochem*, 103, 281-285 (1988)]. The DNA thus produced is fragmented with a suitable restriction endonuclease, and DNA fragments thus obtained are ligated with a cloning vector to transform the microorganism. A plasmid DNA is extracted from the transformant and treated with a restriction endonuclease to produce a DNA fragment containing 1404 bases encoding the thrombin-binding substance derived from human urine. An oligonucleotide having a nucleotide sequence encoding an amino acid sequence, $X_1X_2Y_1$SerGlySerGly$Y_2$, is inserted into the DNA fragment, thus obtaining a DNA fragment which contains the DNA fragment of the present invention. Typical examples of DNA fragments of the present invention are those having a nucleotide sequence of SEQ ID No. 3 and SEQ ID No. 4. The DNA fragments of the present invention, however, are not limited to them. Any DNA fragments capable of encoding an amino acid sequence constituting the thrombin-binding substance which is the target of the present invention, i.e., the Sequence A, preferably SEQ ID No. 1 and SEQ ID No. 2, are included in the present invention.

The construction of the recombinant vector containing the DNA fragment of the present invention may be carried out by connecting the DNA fragment of the present invention with a replicable expression vector.

As the expression vector, those from any sources, e.g., procaryotes (typically *E. coli*), yeasts, insect viruses, vertebrate viruses, etc., can be used, so long as they are replicable.

In order to ensure efficient production of the thrombin-binding substance, it is desirable that the recombinant expression vector be constructed from the following nucleotide sequences (1) to (7) in this order toward the downstream direction of the transcription.

(1) A nucleotide sequence acting as a promoter.
(2) A nucleotide sequence functioning as a ribosome binding site.
(3) A nucleotide sequence acting as a initiation codon.
(4) A nucleotide sequence encoding a signal peptide.
(5) A nucleotide sequence encoding the amino acid sequence of Sequence (A).
(6) A nucleotide sequence acting as a termination codon.
(7) A nucleotide sequence acting as a poly A addition signal.

A plasmid DNA is preferably used as a vector, for instance, a plasmid which can multiply itself, e.g., in *E. coli* as a host microorganism, and can express the inserted gene by transforming mammalian cells. Such a plasmid DNA comprises nucleotide sequences required for the plasmid to multiply itself in *E. coli*, such as a nucleotide sequence acting as a replicator of ColEI plasmid series, a nucleotide sequence acting as a promoter in mammalian cells, a gene functioning as a selection marker of the transformed *E. coli*, and a gene functioning as a selection marker of the transformed mammalian cells. In a preferable embodiment, it further include a replicator nucleotide sequence such as SV40 ori, polyoma ori, or HSV ori which functions in mammalian cells. Given as preferable examples of promoters are promoters, e.g., cytomegalovirus, SV40, polyoma virus, bovine papilloma virus, adenovirus, etc; retrovirus LTR, e.g., MMTV; a promoter of metallothionein gene, and the like. Examples of *E. coli* selection markers are ampicillin resistant genes, kanamycin resistant genes, tetracycline resistant genes, chloramphenicol resistant genes, and the like. Given as examples of mammalian cell selection markers are neomycin resistant genes, hygromycin B resistant genes, thymidine kinase genes, dihydrofolate reductase genes, xanthine-guanine phosphoribosyl transferase genes, and the like. These genes can be used either singly or in combination of two or more.

Incorporation of the DNA fragment of the present invention into the above vectors can be carried out by cutting a DNA containing the DNA fragment with a suitable restriction endonuclease, optionally, adding a suitable linker, and combining it with the vector which is cut by a suitable restriction endonuclease. Restriction endonucleases which can be used here are, for example, *Eco* RI, *Sph* I, *Pst* I, *Hind* III, *Bam* HI, *Xho* I, *Xba* I, *Ban* III, *Sma* I, *Nco* I, and the like. Nucleotide modification enzymes such as exonuclease III, Bal31, SI nuclease, exonuclease VII, mungbean nuclease, DNA polymerase, and the like can also be used. As a linker, *Eco* RI linker, *Sma* I linker, *Nco* I linker, *Bam* HI linker, *Xho* I linker, *Hind* III linker, *Pst* I linker, *Sph* I linker, *XbaI* I linker, or the like may be used.

Transformed cells which can efficiently produce the recombinant vector and/or thrombin-binding substance of the present invention can be obtained by introducing the expression recombinant vector obtained by the above method into host cells by means of the competent cell method, the protoplast method, the calcium phosphate coprecipitation method, the electroporation method, the DEAE dextran method, the lipofectin method, or the like. Unicellular organisms, such as bacteria and yeasts, cultured insect cells, cultured vertebrate cells, and the like are preferably used as host cells for obtaining the transformant. Various mutants of *E. coli* K12 strain, e.g., HB101, C600K, JM101, JM103, JM105, JM109, MV1034, MV1184, MC1061/P3, and the like, are preferably used as *E. coli* host cells. Preferable examples given of mammalian cells are COS cells, CHO cells, L cells, C127 cells, NIH3T3 cells, HeLa cells, and the like.

The thrombin-binding substance can be obtained by cultivating the transformant thus obtained, extracting and separating it from the cultivated cells or the culture broth. Various natural or artificial media can be used for the cultivation of the transformed cells. The media preferably contain carbon sources such as sugars, alcohols, and salts of organic acids; nitrogen sources such as protein mixtures, amino acids, and ammonium salts; and inorganic salts. In addition, vitamins and antibiotics corresponding to the selection marker genes may preferably be included. If the vector is of the type of which the expression can be controlled, it is necessary to add a procedure for inducing the expression in the course of the cultivation. After the cultivation, the culture broth is centrifuged to separate culture liquid from the cells. In the case where the thrombin-binding substance accumulates in the cultured cells, the cells are destroyed by means of freeze-thaw, ultrasonic treatment, French press, enzyme treatment, homogenizing, or the like, and the thrombin-binding substance is dissolved by using EDTA, surfactants, urea, guanidine hydrochloride, or the like.

A purified thrombin-binding substance can be obtained by submitting the culture liquid or the cell extract containing the thrombin-binding substance thus prepared to column chromatography. Ion-exchange chromatography, affinity chromatography, e.g., that using the monoclonal antibody described in Japanese Patent Laid-open (kokai) No. 45398/1989, gel filtration chromatography, or the like can be used either independently or in combination. Among the thrombin-binding substances thus obtained those having the amino acid sequence of SEQ ID No. 1 or SEQ ID No. 2 possess the following characteristic.

(1) Amino acid sequence:

Based on the nucleotide sequence of the DNA fragments, the amino acid sequence is considered to be those shown in SEQ ID Nos. 1 and 2.

(2) Molecular weight:

55,000-100,000 determined by the SDS-polyacrylamide gel electrophoresis under nonreduced conditions.

(3) Isoelectric point:

pH 3-4 determined by the isoelectric electrophoresis method using ampholite.

(4) Sugar analysis:

Two or more sugars are considered to be attached to the thrombin-binding substances from the molecular weight. Based on the amino acid sequence, one of the sugars is considered to be an acidic polysaccharide attached to Ser (474).

(5) Actions:

Possesses antithrombin activity.
Increases the activity of the antithrombin III.
Possesses platelet aggregation inhibitory activity.

Injection preparations are typical examples of the composition comprising the thrombin-binding substance of the present invention as an anticoagulant agent. A preferable form of such injection preparations is a freeze-dried powder which can be dissolved into distilled water or physiological saline each time it is administered. Intravenous injection is a preferable manner by which the preparation is administered.

Although a dose depends on the symptoms of the patient, the body weight, and the like, a preferable dose is 10 $\mu$g/kg to 10 mg/kg. The thrombin-binding substance of the present invention induces no abnormality with the dose of the above range. It is a quite safe substance.

Other features of the invention will become apparent in the course of the following description of the exemplary embodiments which are given for illustration of the invention and are not intended to be limiting thereof.

## EXAMPLES

Example 1 〈Cloning of thrombin-binding substance gene〉

Primer #1 having the sequence of SEQ ID No. 5 and primer #2 having the sequence of SEQ ID No. 6 were synthesized by using a DNA synthesizer (ABI Model 381A) referring to the nucleotide sequence of human thrombomodulin gene [Shirai, T et al, *J. Biochem*, 103, 281-285 (1988)]. A template DNA was prepared by digesting a human placenta genome DNA (a product of Clonetech Co.) with *Bam* HI. The gene amplification was carried out in the reaction solution of the following formulation using Quick Thermo System (Model QTS-10M: trademark, manufactured by Japan Genetic Co.) by the repetition of 30 cycles of incubation; one cycle consisted of incubation at 94°C for 2 minutes, at 50°C for 3 minutes, and at 72°C for 4 minutes. After the reaction, a portion of the reaction product was sampled to confirm amplification of the target DNA band by the agarose gel electrophoresis.

| 〈Reaction Solution〉 | |
|---|---|
| Distilled water | 71 µl |
| Buffer solution * | 10 µl |
| dNTP mixed solution (2.5 mM) | 8 µl |
| Primer #1 (20 µM) | 5 µl |
| Primer #2 (20 µM) | 5 µl |
| Template DNA (1 µg/µl) | 1 µl |
| AmpliTaq (5 units/µl) | 0.5 µl |

* Buffer solution:
0.1 M potassium chloride
0.1 M Tris-HCl buffer (pH 8.3)
0.1% gelatin
15 mM magnesium chloride

DNA was collected from the reaction solution by the ethanol precipitation, digested with *Xho* I and *Kpn* I and subjected to the agarose gel electrophoresis to obtain 1.57 kb *Xho* I-*Kpn* I fragments. Separately, the vector for the cloning pUC118 [Vieira, J. and Messing, J., *Methods Enzymol.*, 153, 3-11 (1987)] was digested with *Hind* II, connected with *Xho* I linker, and further digested with *Xho* I and *Kpn* I to obtain vector fragments by the agarose gel electrophoresis. The vector fragments and the 1.57 kb *Xho* I-*Kpn* I fragments were ligated and *E. coli* MV1034 [Vieira, J. and Messing, J., *Methods Enzymol.*, 153, 3-11 (1987)] was transformed with the ligated DNA.

Plasmid DNA was extracted from the transformant thus obtained and digested with restriction endonuclease. In this manner, 6 clones holding a plasmid to which the 1.57 kb *Xho* I-*Kpn* I fragment derived from human thrombomodulin gene was inserted were selected.

The determination of nucleotide sequences of the inserted fragments in clones thus obtained revealed 1 to 3 mutated sites in each fragment. Then, 0.31 kb *Xho* I-*Sma* I fragment from clone 2, 0.65 kb *Sma* I-*Mlu* I fragment from clone 1, and 0.62 kb *Mlu* I-*Kpn* I fragment from clone 4, all without mutated sites, were recombined with the abovementioned vector fragment to obtain plasmid pUCTM/XHO-KPN containing an inserted fragment of the human thrombomodulin gene with the correct sequence.

Example 2 〈Construction of the vector for the expression of thrombin-binding substance〉

In order to combine a glycosaminoglycan addition site to Asp at C-terminal of the amino acid sequence of the thrombin-binding substance derived from human urine, linkers $1 to $6 with the nucleotide sequences of SEQ ID Nos. 7 to 12, respectively, were synthesized and each 5'-end was phosphorylated.

The pUCTM/XHO-KPN was digested with *Xho* I and *Kpn* I to prepare a 1.57 kb *Xho* I-*Kpn* I fragment derived from a human thrombomodulin gene. This 1.57 kb fragment was ligated with a mammalian cell expression vector CDM8 (a product of Invitrogen Co.) which had been digested with *Xho* I and dephosphorylated together with linkers $1, $2, $3, and $4. The 1.57 kb fragment was also ligated with *Xho* I digested and dephosphorylated CDM8 with linkers $1, $2, $5, and $6. *E. coli* MC1061/P3 [Seed, B. and Aruffo, A., Proc. Natl. Acad. Sci., USA, 84, 3365-3369 (1987)] was transformed with the ligated DNAs. Plasmid DNAs were extracted from the transformants thus prepared and digested with restriction endonucleases to confirm the direction and the site of the insertion. 1.68 kb fragments containing the DNA fragment of the present invention were cut out by *Xho* I from 8 clones which showed the correct direction of insertion and the correct restriction endonuclease map. The nucleotide sequences of all clones were found to have the sequence of SEQ ID No. 13 or 14, confirming that the expression vectors were correctly constructed.

The expression vector of the present invention thus obtained were named pCDM-GAG-UTM1 and pCDM-GAG-UTM2 (Figure 1), and the transformant harboring the vectors were named *E. coli* MC1061/P3 (pCDM-GAG-UTM1) and *E. coli* MC1061/P3 (pCDM-GAG-UTM2).

Example 3 〈Expression of the thrombin-binding substance by the cultured mammalian cells〉

COS7 cells were transfected with pCDM-GAG-UTM1 or pCDM-GAG-UTM2 by the DEAE-Dextran method [Seed, B. and Aruffo, A., Proc. Natl. Acad. Sci., USA, 84, 3365-3369 (1987)]. $5x10^5$ cells were inoculated into a 60 mm culture dish and, on the next day, the culture medium was aspirated and replaced by 2 ml of Dulbecco's-modified minimum essential medium (DMEM) containing 10% Nu-serum. 10 µg (1 µg/µl) of pCDM-GAG-UTM1 or pCDM-GAG-UTM2 were added to 100 µl of a 10 mg/ml DEAE-Dextran solution (average molecular weight: $5x10^5$, a product of Pharmacia) in PBS, and the resulting solution was added to cell culture liquid together with 10 µl of 20 mM chloroquine. After cultivating for 4 hours at 37°C, the culture medium was aspirated and 2 ml of 10% DMSO (dissolved in PBS) was added. The mixture was allowed to stand still at room temperature for 2 minutes. After removal of the DMSO solution by aspiration, 3 ml of DMEM containing 10% FCS was added and the mixture was cultivated at 37°C for 24 hours. The culture medium was replaced by DMEM containing no FCS, followed by continued cultivation for a further 48 hours. After the cultivation, the supernatant was collected.

The culture medium obtained by the above procedure was passed through a 1 ml Sepharose 4B (2 mg IgG/ml resin) column with which monoclonal antibody A-73 (Japanese Patent Laid-open (kokai) No. 45398/1989; 2 mg IgG/ml resin) was combined. The column was washed with (1) 2 ml of 0.02 M Tris-HCl buffer (pH 7.4) containing 0.1 M NaCl, (2) 20 ml of 0.02 M Tris-HCl buffer (pH 7.4) containing 1 M NaCl and0.05% Tween 20, and (3) 5 ml of 0.02 M Tris-HCl buffer (pH 7.4) containing 1 M NaCl, followed by elution with 5 ml of 0.02 M Tris-HCl buffer (pH 7.4) containing 2 M sodium thiocyanate, 5 mM EDTA, and 1 M NaCl. The eluate was dialyzed against 50 mM acetate buffer containing 0.1 M NaCl (pH 4.5) and applied on a column of Mono-Q sepharose. The column was washed with the same buffer and eluted with linear gradient of 0.1 to 2 M NaCl in 50 mM acetate buffer (pH 4.5) to obtain purified thrombin-binding substances (r-GAG-UTM1 and r-GAG-UTM2).

Example 4 〈Expression of the thrombin-binding substance by the cultured mammalian cells〉

CHO•K1 cells were transfected with pCDM-GAG-UTM1 by the calcium phosphate method [Gorman, C., "DNA Cloning" IRL Press, England, vol. 2, 143-190 (1985)]. $5x10^5$ CHO•K1 cells were inoculated into a 10 cm petri dish and, on the next day, the culture medium (Ham F12 medium containing 10% FCS, hereinafter referred to as Medium) was exchanged. Four (4) hours thereafter, a coprecipitate of DNA and calcium phosphate was added. The coprecipitate used here was prepared according to the following manner. 20 µg of pCDM-GAG-UTM1 and 100 ng of neomycin resistant gene dissolved into 450 µl of 1 mM Tris-HCl buffer (pH 8.0)-0.1 mM EDTA and mixed with 50 µl of 2.5 M calcium chloride. The mixture was added dropwise to 500 µl of solution: 50 mM HEPES (pH 7.12)-280 mM NaCl-1.5 mM sodium hydrogen phosphate, and after allowing to stand still, the solution was added to the cell culture medium for cultivation for 24 hours. The medium was replaced by a fresh one and cultivated for a further 24 hours, following which the medium was replaced by a selective medium containing 400 µg/ml G418. After 2 weeks, colonies produced were transferred to a 24-well plate and continuously cultivated up to the confluent. The supernatant was collected from the culture broth. The secreted thrombin-binding substance (r-GAG-UTM1) was quantitatively analyzed to select high producing clones. The cloning was further carried out on the selected clone by the limiting dilution method. The transformed cells thus obtained was named CHO-GUTM 1-8 and deposited with Fermentation Research Institute, Agency of Industrial Science and Technology (FERM P-3260).

The transformed cell CHO-GUTM 1-8 was cultured in UC202 medium (a product of Nissui Pharmaceutical Co.) containing 1% FCS in a 225 $cm^2$ flask to become confluent, following which the medium was replaced by 50 ml of UC202 medium without containing FCS. After 1 week, the culture supernatant was collected and the same amount of the fresh medium not containing FCS was added. After the cultivation of a further 1 week, the culture supernatant was collected and confirmed to contain 3-4 µg/ml thrombin-binding substance therein secreted.

The purified thrombin-binding substance was obtained according to the same procedure of the later part of Example 3.

Example 5 ⟨Characteristics of thrombin-binding substance⟩

The SDS-PAGE was performed according to the Laemmli's method (*Nature*, 227, 680-685) on the purified thrombin-binding substances. The protein was transferred onto a PVDF membrane according to the Matsudaira's method [*J. Biol. Chem.*, *262* (*21*), 10035-10038]. The PVDF membrane was then reacted in 0.05 M Tris-HCl buffer (TBS) containing 0.1% bovine serum albumin and 0.1 M NaCl at room temperature for 2 hours. After discharging the solution, the residue was washed thoroughly with a TBS-0.05% Tween 20, reacted with horseradish peroxidase conjugated monoclonal antibody A-60 in TBS-0.05% Tween 20 solution at room temperature for 1 hour. The solution was discharged, and the residue was washed thoroughly with a 0.05% Tween 20-TBS and put into 50 ml of an acetic acid buffer (pH 5.0) containing 5 mg of 3-amino-9-ethylcarbazole and 25 µl of 30% hydrogen peroxide to get colored to confirm a broad band which is characteristic to glycosaminoglycan adducts.

Example 6

r-UTM and r-GAG-UTM1 and 2 which are the thrombin-binding substances of the present invention, 0.1 µg/ml each, were treated with 5 µl of chondroitinase (10 mU, a product of Seikagaku Kogyo K.K.) at 37°C for 40 minutes. The immunoblotting was carried out in the same manner as in Example 5 to confirm the presence of chondroitin sulfate type glycosaminoglycan covalent bonds in the thrombin-binding substances of the present invention.

Example 7 ⟨Anti-coagulant activity⟩

r-UTM and r-GAG-UTM1 and 2 of the thrombin-binding substance of the present invention, 2.5 µg/ml each, were mixed with human fibrinogen (2.5 mg/ml) and human antithrombin III (0 or 250 µg/ml), and dissolved in 5 mM solution of $CaCl_2$. Bovine thrombin (0.5 U/ml) was added to the solutions to measure the clotting time. The results are shown in Table 1.

TABLE 1

| | Control (sec.) | r-UTM (sec.) | r-GAG-UTM1 (sec.) | r-GAG-UTM2 (sec.) |
|---|---|---|---|---|
| ATIII (-) | 43.3 | 61.8 | 77.2 | 80.1 |
| ATIII (+) | 49.5 | 80.8 | >400 | >400 |

Table I demonstrates that the thrombin-binding substances of the present invention delay blood coagulation by combining with thrombin. A remarkable promotion of the anti-coagulant activity of the thrombin-binding substances by the presence of antithrombin III are also shown.

Example 8 ⟨Anti-coagulant activity⟩

r-UTM (9-90 nM), r-GAG-UTM1, or r-GAG-UTM2 of the thrombin-binding substance of the present invention (9-90 nM), dissolved in a solution of bovine fibrinogen (1 mg/ml) in 20 mM Tris-HCl buffer (pH 7.4) containing 0.15 M NaCl, was mixed with bovine thrombin (18 nM) to measure the time required for the coagulation. 50% inhibitory concentrations ($IC_{50}$) were determined from the calibration curve prepared by using bovine thrombin of various concentrations. The results are shown in Table 2.

TABLE 2

| | $IC_{50}$ (nM) |
|---|---|
| r-UTM | 80 |
| r-GAG-UTM1 | 16 |
| r-GAG-UTM2 | 15 |

Example 9 ⟨Anti-coagulant activity⟩

Substances of the present invention (17 nM) or r-UTM (17 nM), dissolved in a solution of bovine fibrinogen (1 mg/ml) in 20 mM Tris-HCl buffer (pH 7.4) containing 0.15 M NaCl, was mixed with bovine thrombin (18 nM) to measure the time required for the coagulation. The results are shown in Table 3.

TABLE 3

| | Coagulation time (sec) |
|---|---|
| Control | 28.1 |
| r-UTM | 29.6 |
| r-GAG-UTM1 | 300.0 |
| r-GAG-UTM2 | 295.3 |

Example 10 ⟨Platelet aggregation inhibitory activity⟩

To 8 μl of a solution of a substance of the present invention ($10^{-6}$-$10^{-8}$ M) and platelet rich plasma (PRP) (200 μl), prepared from blood taken from rabbit ear vein, was added 2μM adenosine diphosphate (ADP) to measure the platelet aggregation. 50% inhibitory concentration, i.e., the concentration of the compounds of the present invention to inhibit ADP aggregation, determined based on the calibration curve which was prepared by using ADP at various concentrations, were $2x10^{-7}$ M for r-GAG-UTM1 and $2.1x10^{-7}$ M for r-GAG-UTM2. r-UTM exhibited no aggregation inhibitory activity within the tested concentration range ($10^{-6}$-$10^{-8}$ M).

Example 11 ⟨Changes in Blood Concentration⟩

A catheter was inserted into the right femoral vein of Wistar rats (male) under anesthesia, and through the catheter were rapidly administered 1 mg/ml/kg of the tested compounds, r-GAG-UTM1 and r-UTM. Blood samples, 0.1 ml each, taken before the administration and 1, 3, 6, 10, 20, 30, 60, and 120 minutes after the administration were mixed with heparin and served as plasma samples for the determination of the blood concentration. The measurement of the blood concentration was performed according to the sandwich ELISA method using an anti-human thrombin-binding monoclonal antibody. The both tested compounds were found to be analyzable with the one-compartment model. The results are shown in the following Table.

TABLE 4

| | r-GAG-UTM1 (n = 3) | r-UTM (n = 5) |
|---|---|---|
| $T_{1/2}$ (min) | 75.2±10.8 | 45.4±2.6 |
| AUC (min•μg/ml) | 1380±61 | 872±64 |

As illustrated above thrombin-binding substances of the present invention promote antithrombin III activity and inhibit platelet aggregation, and by themselves possess antithrombin activity. Thus, they are useful as an effective component of anticoagulant agents. Furthermore, the thrombin-binding substance of the present invention can be produced inexpensively in a large scale.

Obviously, numerous modifications and variations of the present invention are possible in light of the above teachings. It is therefore to be understood that within the scope of the appended claims, the invention may be practiced otherwise than as specifically described herein.

SEQUENCE LISTINGS

1. SEQ ID No. 1

2. Length of the sequence: 476

3. Type of the sequence: Amino acid

4. Topology of the sequence: Linear

5. Kind: Protein

6. Sequence

```
Ala Pro Ala Glu Pro Gln Pro Gly Gly Ser Gln Cys Val Glu His Asp
                5                   10                  15

Cys Phe Ala Leu Tyr Pro Gly Pro Ala Thr Phe Leu Asn Ala Ser Gln
            20                  25                  30

Ile Cys Asp Gly Leu Arg Gly His Leu Met Thr Val Arg Ser Ser Val
        35                  40                  45

Ala Ala Asp Val Ile Ser Leu Leu Leu Asn Gly Asp Gly Gly Val Gly
    50                  55                  60

Arg Arg Arg Leu Trp Ile Gly Leu Gln Leu Pro Pro Gly Cys Gly Asp
65                  70                  75                  80

Pro Lys Arg Leu Gly Pro Leu Arg Gly Phe Gln Trp Val Thr Gly Asp
                85                  90                  95

Asn Asn Thr Ser Tyr Ser Arg Trp Ala Arg Leu Asp Leu Asn Gly Ala
            100                 105                 110

Pro Leu Cys Gly Pro Leu Cys Val Ala Val Ser Ala Ala Glu Ala Thr
        115                 120                 125

Val Pro Ser Glu Pro Ile Trp Glu Glu Gln Gln Cys Glu Val Lys Ala
    130                 135                 140

Asp Gly Phe Leu Cys Glu Phe His Phe Pro Ala Thr Cys Arg Pro Leu
145                 150                 155                 160

Ala Val Glu Pro Gly Ala Ala Ala Ala Ala Val Ser Ile Thr Tyr Gly
                165                 170                 175

Thr Pro Phe Ala Ala Arg Gly Ala Asp Phe Gln Ala Leu Pro Val Gly
            180                 185                 190
```

Ser Ser Ala Ala Val Ala Pro Leu Gly Leu Gln Leu Met Cys Thr Ala
195 200 205

Pro Pro Gly Ala Val Gln Gly His Trp Ala Arg Glu Ala Pro Gly Ala
210 215 220

Trp Asp Cys Ser Val Glu Asn Gly Gly Cys Glu His Ala Cys Asn Ala
225 230 235 240

Ile Pro Gly Ala Pro Arg Cys Gln Cys Pro Ala Gly Ala Ala Leu Gln
245 250 255

Ala Asp Gly Arg Ser Cys Thr Ala Ser Ala Thr Gln Ser Cys Asn Asp
260 265 270

Leu Cys Glu His Phe Cys Val Pro Asn Pro Asp Gln Pro Gly Ser Tyr
275 280 285

Ser Cys Met Cys Glu Thr Gly Tyr Arg Leu Ala Ala Asp Gln His Arg
290 295 300

Cys Glu Asp Val Asp Asp Cys Ile Leu Glu Pro Ser Pro Cys Pro Gln
305 310 315 320

Arg Cys Val Asn Thr Gln Gly Gly Phe Glu Cys His Cys Tyr Pro Asn
325 330 335

Tyr Asp Leu Val Asp Gly Glu Cys Val Glu Pro Val Asp Pro Cys Phe
340 345 350

Arg Ala Asn Cys Glu Tyr Gln Cys Gln Pro Leu Asn Gln Thr Ser Tyr
355 360 365

Leu Cys Val Cys Ala Glu Gly Phe Ala Pro Ile Pro His Glu Pro His
370 375 380

Arg Cys Gln Met Phe Cys Asn Gln Thr Ala Cys Pro Ala Asp Cys Asp
385 390 395 400

Pro Asn Thr Gln Ala Ser Cys Glu Cys Pro Glu Gly Tyr Ile Leu Asp
405 410 415

Asp Gly Phe Ile Cys Thr Asp Ile Asp Glu Cys Glu Asn Gly Gly Phe
420 425 430

Cys Ser Gly Val Cys His Asn Leu Pro Gly Thr Phe Glu Cys Ile Cys
435 440 445

Gly Pro Asp Ser Ala Leu Val Arg His Ile Gly Thr Asp Cys Asp Ser
450 455 460

Gly Lys Val Asp Glu Asp Tyr Ser Gly Ser Gly Glu
465 470 475

1. SEQ ID No. 2
2. Length of the sequence: 476
3. Type of the sequence: Amino acid
4. Topology of the sequence: Linear
5. Kind: Protein
6. Sequence

```
Ala Pro Ala Glu Pro Gln Pro Gly Gly Ser Gln Cys Val Glu His Asp
                  5                  10                  15

Cys Phe Ala Leu Tyr Pro Gly Pro Ala Thr Phe Leu Asn Ala Ser Gln
             20                  25                  30

Ile Cys Asp Gly Leu Arg Gly His Leu Met Thr Val Arg Ser Ser Val
         35                  40                  45

Ala Ala Asp Val Ile Ser Leu Leu Leu Asn Gly Asp Gly Gly Val Gly
     50                  55                  60

Arg Arg Arg Leu Trp Ile Gly Leu Gln Leu Pro Pro Gly Cys Gly Asp
 65                  70                  75                  80

Pro Lys Arg Leu Gly Pro Leu Arg Gly Phe Gln Trp Val Thr Gly Asp
                 85                  90                  95

Asn Asn Thr Ser Tyr Ser Arg Trp Ala Arg Leu Asp Leu Asn Gly Ala
            100                 105                 110

Pro Leu Cys Gly Pro Leu Cys Val Ala Val Ser Ala Ala Glu Ala Thr
        115                 120                 125

Val Pro Ser Glu Pro Ile Trp Glu Glu Gln Gln Cys Glu Val Lys Ala
    130                 135                 140

Asp Gly Phe Leu Cys Glu Phe His Phe Pro Ala Thr Cys Arg Pro Leu
145                 150                 155                 160

Ala Val Glu Pro Gly Ala Ala Ala Ala Ala Val Ser Ile Thr Tyr Gly
                165                 170                 175

Thr Pro Phe Ala Ala Arg Gly Ala Asp Phe Gln Ala Leu Pro Val Gly
            180                 185                 190

Ser Ser Ala Ala Val Ala Pro Leu Gly Leu Gln Leu Met Cys Thr Ala
        195                 200                 205
```

```
Pro Pro Gly Ala Val Gln Gly His Trp Ala Arg Glu Ala Pro Gly Ala
    210                     215                     220

Trp Asp Cys Ser Val Glu Asn Gly Gly Cys Glu His Ala Cys Asn Ala
225                 230                     235                 240

Ile Pro Gly Ala Pro Arg Cys Gln Cys Pro Ala Gly Ala Ala Leu Gln
                245                     250                     255

Ala Asp Gly Arg Ser Cys Thr Ala Ser Ala Thr Gln Ser Cys Asn Asp
            260                     265                     270

Leu Cys Glu His Phe Cys Val Pro Asn Pro Asp Gln Pro Gly Ser Tyr
        275                     280                     285

Ser Cys Met Cys Glu Thr Gly Tyr Arg Leu Ala Ala Asp Gln His Arg
    290                     295                     300

Cys Glu Asp Val Asp Asp Cys Ile Leu Glu Pro Ser Pro Cys Pro Gln
305                 310                     315                     320

Arg Cys Val Asn Thr Gln Gly Gly Phe Glu Cys His Cys Tyr Pro Asn
                325                     330                     335

Tyr Asp Leu Val Asp Gly Glu Cys Val Glu Pro Val Asp Pro Cys Phe
            340                     345                     350

Arg Ala Asn Cys Glu Tyr Gln Cys Gln Pro Leu Asn Gln Thr Ser Tyr
        355                     360                     365

Leu Cys Val Cys Ala Glu Gly Phe Ala Pro Ile Pro His Glu Pro His
    370                     375                     380

Arg Cys Gln Met Phe Cys Asn Gln Thr Ala Cys Pro Ala Asp Cys Asp
385                 390                     395                     400

Pro Asn Thr Gln Ala Ser Cys Glu Cys Pro Glu Gly Tyr Ile Leu Asp
                405                     410                     415

Asp Gly Phe Ile Cys Thr Asp Ile Asp Glu Cys Glu Asn Gly Gly Phe
            420                     425                     430

Cys Ser Gly Val Cys His Asn Leu Pro Gly Thr Phe Glu Cys Ile Cys
        435                     440                     445

Gly Pro Asp Ser Ala Leu Val Arg His Ile Gly Thr Asp Cys Asp Ser
    450                     455                     460

Gly Lys Val Asp Asp Glu Ala Ser Gly Ser Gly Asp
465                 470                     475
```

1. SEQ ID No. 3
2. Length of the sequence: 1428
3. Type of the sequence: Nucleic acid
4. Strandedness: Double-stranded
5. Topology of the sequence: Linear
6. Kind: cDNA to mRNA
7. Sequence

```
GCACCCGCAG AGCCGCAGCC GGGTGGCAGC CAGTGCGTCG AGCACGACTG CTTCGCGCTC   60
TACCCGGGCC CCGCGACCTT CCTCAATGCC AGTCAGATCT GCGACGGACT GCGGGGCCAC  120
CTAATGACAG TGCGCTCCTC GGTGGCTGCC GATGTCATTT CCTTGCTACT GAACGGCGAC  180
GGCGGCGTTG GCCGCCGGCG CCTCTGGATC GGCCTGCAGC TGCCACCCGG CTGCGGCGAC  240
CCCAAGCGCC TCGGGCCCCT GCGCGGCTTC CAGTGGGTTA CGGGAGACAA CAACACCAGC  300
TATAGCAGGT GGGCACGGCT CGACCTCAAT GGGGCTCCCC TCTGCGGCCC GTTGTGCGTC  360
GCTGTCTCCG CTGCTGAGGC CACTGTGCCC AGCGAGCCGA TCTGGGAGGA GCAGCAGTGC  420
GAAGTGAAGG CCGATGGCTT CCTCTGCGAG TTCCACTTCC CAGCCACCTG CAGGCCACTG  480
GCTGTGGAGC CCGGCGCCGC GGCTGCCGCC GTCTCGATCA CCTACGGCAC CCCGTTCGCG  540
GCCCGCGGAG CGGACTTCCA GGCGCTGCCG GTGGGCAGCT CCGCCGCGGT GGCTCCCCTC  600
GGCTTACAGC TAATGTGCAC CGCGCCGCCC GGAGCGGTCC AGGGGCACTG GGCCAGGGAG  660
GCGCCGGGCG CTTGGGACTG CAGCGTGGAG AACGGCGGCT GCGAGCACGC GTGCAATGCG  720
ATCCCTGGGG CTCCCCGCTG CCAGTGCCCA GCCGGCGCCG CCCTGCAGGC AGACGGGCGC  780
TCCTGCACCG CATCCGCGAC GCAGTCCTGC AACGACCTCT GCGAGCACTT CTGCGTTCCC  840
AACCCCGACC AGCCGGGCTC CTACTCGTGC ATGTGCGAGA CCGGCTACCG GCTGGCGGCC  900
GACCAACACC GGTGCGAGGA CGTGGATGAC TGCATACTGG AGCCCAGTCC GTGTCCGCAG  960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
```

CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG 1428

1. SEQ ID No. 4
2. Length of the sequence: 1428
3. Type of the sequence: Nucleic acid
4. Strandedness: Double-stranded
5. Topology of the sequence: Linear
6. Kind: cDNA to mRNA
7. Sequence

GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780

```
TCCTGCACCG CATCCGCGAC GCAGTCCTGC AACGACCTCT GCGAGCACTT CTGCGTTCCC   840
AACCCCGACC AGCCGGGCTC CTACTCGTGC ATGTGCGAGA CCGGCTACCG GCTGGCGGCC   900
GACCAACACC GGTGCGAGGA CGTGGATGAC TGCATACTGG AGCCCAGTCC GTGTCCGCAG   960
CGCTGTGTCA ACACACAGGG TGGCTTCGAG TGCCACTGCT ACCCTAACTA CGACCTGGTG  1020
GACGGCGAGT GTGTGGAGCC CGTGGACCCG TGCTTCAGAG CCAACTGCGA GTACCAGTGC  1080
CAGCCCCTGA ACCAAACTAG CTACCTCTGC GTCTGCGCCG AGGGCTTCGC GCCCATTCCC  1140
CACGAGCCGC ACAGGTGCCA GATGTTTTGC AACCAGACTG CCTGTCCAGC CGACTGCGAC  1200
CCCAACACCC AGGCTAGCTG TGAGTGCCCT GAAGGCTACA TCCTGGACGA CGGTTTCATC  1260
TGCACGGACA TCGACGAGTG CGAAAACGGC GGCTTCTGCT CCGGGGTGTG CCACAACCTC  1320
CCCGGTACCT TCGAGTGCAT CTGCGGGCCC GACTCGGCCC TTGTCCGCCA CATTGGCACC  1380
GACTGTGACT CCGGCAAGGT CGACGACGAG GCCAGCGGCT CTGGCGAC               1428
```

1. SEQ ID No. 5
2. Length of the sequence: 21
3. Type of the sequence: Nucleic acid
4. Strandedness: Single-stranded
5. Topology of the sequence: Linear
6. Kind: other nucleic acid, synthesized DNA
7. Sequence

AGGGCCGGGC ACTTATAAAC T

1. SEQ ID No. 6
2. Length of the sequence: 21
3. Type of the sequence: Nucleic acid
4. Strandedness: Single-stranded
5. Topology of the sequence: Linear

6. Kind: other nucleic acid, synthesized DNA
7. Sequence

CCCAGTGGTC CAGTGACGTC A

1. SEQ ID No. 7
2. Length of the sequence: 39
3. Type of the sequence: Nucleic acid
4. Strandedness: Single-stranded
5. Topology of the sequence: Linear
6. Kind: other nucleic acid, synthesized DNA
7. Sequence

CTTCGAGTGC ATCTGCGGGC CCGACTCGGC CCTTGTCCG

1. SEQ ID No. 8
2. Length of the sequence: 49
3. Type of the sequence: Nucleic acid
4. Strandedness: Single-stranded
5. Topology of the sequence: Linear
6. Kind: other nucleic acid, synthesized DNA
7. Sequence

ATGTGGCGGA CAAGGGCCGA GTCGGGCCCG CAGATGCACT CGAAGGTAC

1. SEQ ID No. 9
2. Length of the sequence: 65
3. Type of the sequence: Nucleic acid
4. Strandedness: Single-stranded
5. Topology of the sequence: Linear
6. Kind: other nucleic acid, synthesized DNA
7. Sequence

CCACATTGGC ACCGACTGTG ACTCCGGCAA GGTGGACGAG GACTATAGCG GCTCTGGCGA 60

GTGAC 65

1. SEQ ID No. 10
2. Length of the sequence: 63
3. Type of the sequence: Nucleic acid
4. Strandedness: Single-stranded
5. Topology of the sequence: Linear
6. Kind: other nucleic acid, synthesized DNA
7. Sequence

TCGAGTCACT CGCCAGAGCC GCTATAGTCC TCGTCCACCT TGCCGGAGTC ACAGTCGGTG 60

CCA 63

1. SEQ ID No. 11
2. Length of the sequence: 65
3. Type of the sequence: Nucleic acid
4. Strandedness: Single-stranded
5. Topology of the sequence: Linear

6. Kind: other nucleic acid, synthesized DNA
7. Sequence

CCACATTGGC ACCGACTGTG ACTCCGGCAA GGTCGACGAC GAGGCCAGCG GCTCTGGCGA 60
CTGAC 65

1. SEQ ID No. 12
2. Length of the sequence: 63
3. Type of the sequence: Nucleic acid
4. Strandedness: Single-stranded
5. Topology of the sequence: Linear
6. Kind: other nucleic acid, synthesized DNA
7. Sequence

TCGAGTCAGT CGCCAGAGCC GCTGGCCTCG TCGTCGACCT TGCCGGAGTC ACAGTCGGTG 60
CCA 63

1. SEQ ID No. 13
2. Length of the sequence: 1680
3. Type of the sequence: Nucleic acid
4. Strandedness: Double-stranded
5. Topology of the sequence: Linear
6. Kind: cDNA to mRNA
7. Characteristics of the sequence

   Symbol designating the characteristic: sig peptide

   Sites: 190, 243

   Method for the determination of the characteristic: S

   Symbol designating the characteristic: mat peptide

Sites: 244, 1671

Method for the determination of the characteristic: S

8. Sequence

```
CTCGAGCCCT GGCCGATCCG CATGTCAGAG GCTGCCTCGC AGGGGCTGCG CGCAGCGGCA   60

AGAAGTGTCT GGGCTGGGAC GGACAGGAGA GGCTGTCGCC ATCGGCGTCC TGTGCCCCTC  120

TGCTCCGGCA CGGCCCTGTC GCAGTGCCCG CGCTTTCCCC GGCGCCTGCA CGCGGCGCGC  180

CTGGGTAAC ATG CTT GGG GTC CTG GTC CTT GGC GCG CTG GCC CTG GCC GGC  231
          Met Leu Gly Val Leu Val Leu Gly Ala Leu Ala Leu Ala Gly
          -18         -15                 -10                  -5

CTG GGG TTC CCC GCA CCC GCA GAG CCG CAG CCG GGT GGC AGC CAG TGC     279
Leu Gly Phe Pro Ala Pro Ala Glu Pro Gln Pro Gly Gly Ser Gln Cys
                1               5                   10

GTC GAG CAC GAC TGC TTC GCG CTC TAC CCG GGC CCC GCG ACC TTC CTC     327
Val Glu His Asp Cys Phe Ala Leu Tyr Pro Gly Pro Ala Thr Phe Leu
        15                  20                  25

AAT GCC AGT CAG ATC TGC GAC GGA CTG CGG GGC CAC CTA ATG ACA GTG     375
Asn Ala Ser Gln Ile Cys Asp Gly Leu Arg Gly His Leu Met Thr Val
    30                  35                  40

CGC TCC TCG GTG GCT GCC GAT GTC ATT TCC TTG CTA CTG AAC GGC GAC     423
Arg Ser Ser Val Ala Ala Asp Val Ile Ser Leu Leu Leu Asn Gly Asp
45                  50                  55                  60

GGC GGC GTT GGC CGC CGG CGC CTC TGG ATC GGC CTG CAG CTG CCA CCC     471
Gly Gly Val Gly Arg Arg Arg Leu Trp Ile Gly Leu Gln Leu Pro Pro
                65                  70                  75

GGC TGC GGC GAC CCC AAG CGC CTC GGG CCC CTG CGC GGC TTC CAG TGG     519
Gly Cys Gly Asp Pro Lys Arg Leu Gly Pro Leu Arg Gly Phe Gln Trp
            80                  85                  90

GTT ACG GGA GAC AAC AAC ACC AGC TAT AGC AGG TGG GCA CGG CTC GAC     567
Val Thr Gly Asp Asn Asn Thr Ser Tyr Ser Arg Trp Ala Arg Leu Asp
        95                  100                 105

CTC AAT GGG GCT CCC CTC TGC GGC CCG TTG TGC GTC GCT GTC TCC GCT     615
Leu Asn Gly Ala Pro Leu Cys Gly Pro Leu Cys Val Ala Val Ser Ala
    110                 115                 120

GCT GAG GCC ACT GTG CCC AGC GAG CCG ATC TGG GAG GAG CAG CAG TGC     663
Ala Glu Ala Thr Val Pro Ser Glu Pro Ile Trp Glu Glu Gln Gln Cys
125                 130                 135                 140
```

```
GAA GTG AAG GCC GAT GGC TTC CTC TGC GAG TTC CAC TTC CCA GCC ACC     711
Glu Val Lys Ala Asp Gly Phe Leu Cys Glu Phe His Phe Pro Ala Thr
                145                 150                 155

TGC AGG CCA CTG GCT GTG GAG CCC GGC GCC GCG GCT GCC GCC GTC TCG     759
Cys Arg Pro Leu Ala Val Glu Pro Gly Ala Ala Ala Ala Ala Val Ser
            160                 165                 170

ATC ACC TAC GGC ACC CCG TTC GCG GCC CGC GGA GCG GAC TTC CAG GCG     807
Ile Thr Tyr Gly Thr Pro Phe Ala Ala Arg Gly Ala Asp Phe Gln Ala
        175                 180                 185

CTG CCG GTG GGC AGC TCC GCC GCG GTG GCT CCC CTC GGC TTA CAG CTA     855
Leu Pro Val Gly Ser Ser Ala Ala Val Ala Pro Leu Gly Leu Gln Leu
    190                 195                 200

ATG TGC ACC GCG CCG CCC GGA GCG GTC CAG GGG CAC TGG GCC AGG GAG     903
Met Cys Thr Ala Pro Pro Gly Ala Val Gln Gly His Trp Ala Arg Glu
205                 210                 215                 220

GCG CCG GGC GCT TGG GAC TGC AGC GTG GAG AAC GGC GGC TGC GAG CAC     951
Ala Pro Gly Ala Trp Asp Cys Ser Val Glu Asn Gly Gly Cys Glu His
                225                 230                 235

GCG TGC AAT GCG ATC CCT GGG GCT CCC CGC TGC CAG TGC CCA GCC GGC     999
Ala Cys Asn Ala Ile Pro Gly Ala Pro Arg Cys Gln Cys Pro Ala Gly
            240                 245                 250

GCC GCC CTG CAG GCA GAC GGG CGC TCC TGC ACC GCA TCC GCG ACG CAG    1047
Ala Ala Leu Gln Ala Asp Gly Arg Ser Cys Thr Ala Ser Ala Thr Gln
        255                 260                 265

TCC TGC AAC GAC CTC TGC GAG CAC TTC TGC GTT CCC AAC CCC GAC CAG    1095
Ser Cys Asn Asp Leu Cys Glu His Phe Cys Val Pro Asn Pro Asp Gln
    270                 275                 280

CCG GGC TCC TAC TCG TGC ATG TGC GAG ACC GGC TAC CGG CTG GCG GCC    1143
Pro Gly Ser Tyr Ser Cys Met Cys Glu Thr Gly Tyr Arg Leu Ala Ala
285                 290                 295                 300

GAC CAA CAC CGG TGC GAG GAC GTG GAT GAC TGC ATA CTG GAG CCC AGT    1191
Asp Gln His Arg Cys Glu Asp Val Asp Asp Cys Ile Leu Glu Pro Ser
                305                 310                 315

CCG TGT CCG CAG CGC TGT GTC AAC ACA CAG GGT GGC TTC GAG TGC CAC    1239
Pro Cys Pro Gln Arg Cys Val Asn Thr Gln Gly Gly Phe Glu Cys His
            320                 325                 330

TGC TAC CCT AAC TAC GAC CTG GTG GAC GGC GAG TGT GTG GAG CCC GTG    1287
Cys Tyr Pro Asn Tyr Asp Leu Val Asp Gly Glu Cys Val Glu Pro Val
        335                 340                 345
```

```
GAC CCG TGC TTC AGA GCC AAC TGC GAG TAC CAG TGC CAG CCC CTG AAC    1335
Asp Pro Cys Phe Arg Ala Asn Cys Glu Tyr Gln Cys Gln Pro Leu Asn
    350                 355                 360

CAA ACT AGC TAC CTC TGC GTC TGC GCC GAG GGC TTC GCG CCC ATT CCC    1383
Gln Thr Ser Tyr Leu Cys Val Cys Ala Glu Gly Phe Ala Pro Ile Pro
365                 370                 375                 380

CAC GAG CCG CAC AGG TGC CAG ATG TTT TGC AAC CAG ACT GCC TGT CCA    1431
His Glu Pro His Arg Cys Gln Met Phe Cys Asn Gln Thr Ala Cys Pro
                385                 390                 395

GCC GAC TGC GAC CCC AAC ACC CAG GCT AGC TGT GAG TGC CCT GAA GGC    1479
Ala Asp Cys Asp Pro Asn Thr Gln Ala Ser Cys Glu Cys Pro Glu Gly
            400                 405                 410

TAC ATC CTG GAC GAC GGT TTC ATC TGC ACG GAC ATC GAC GAG TGC GAA    1527
Tyr Ile Leu Asp Asp Gly Phe Ile Cys Thr Asp Ile Asp Glu Cys Glu
        415                 420                 425

AAC GGC GGC TTC TGC TCC GGG GTG TGC CAC AAC CTC CCC GGT ACC TTC    1575
Asn Gly Gly Phe Cys Ser Gly Val Cys His Asn Leu Pro Gly Thr Phe
    430                 435                 440

GAG TGC ATC TGC GGG CCC GAC TCG GCC CTT GTC CGC CAC ATT GGC ACC    1623
Glu Cys Ile Cys Gly Pro Asp Ser Ala Leu Val Arg His Ile Gly Thr
445                 450                 455                 460

GAC TGT GAC TCC GGC AAG GTG GAC GAG GAC TAT AGC GGC TCT GGC GAG    1671
Asp Cys Asp Ser Gly Lys Val Asp Glu Asp Tyr Ser Gly Ser Gly Glu
                465                 470                 475

TGACTCGAG                                                          1680
```

1. SEQ ID No. 14
2. Length of the sequence: 1680
3. Type of the sequence: Nucleic acid
4. Strandedness: Double-stranded
5. Topology of the sequence: Linear
6. Kind: cDNA to mRNA
7. Characteristics of the sequence

   Symbol designating the characteristic: sig peptide

   Sites: 190, 243

Method for the determination of the characteristic: S

Symbol designating the characteristic: mat peptide

Sites: 244, 1671

Method for the determination of the characteristic: S

8. Sequence

```
CTCGAGCCCT GGCCGATCCG CATGTCAGAG GCTGCCTCGC AGGGGCTGCG CGCAGCGGCA    60

AGAAGTGTCT GGGCTGGGAC GGACAGGAGA GGCTGTCGCC ATCGGCGTCC TGTGCCCCTC   120

TGCTCCGGCA CGGCCCTGTC GCAGTGCCCG CGCTTTCCCC GGCGCCTGCA CGCGGCGCGC   180

CTGGGTAAC ATG CTT GGG GTC CTG GTC CTT GGC GCG CTG GCC CTG GCC GGC   231
          Met Leu Gly Val Leu Val Leu Gly Ala Leu Ala Leu Ala Gly
          -18         -15                     -10                 -5

CTG GGG TTC CCC GCA CCC GCA GAG CCG CAG CCG GGT GGC AGC CAG TGC     279
Leu Gly Phe Pro Ala Pro Ala Glu Pro Gln Pro Gly Gly Ser Gln Cys
                1               5                   10

GTC GAG CAC GAC TGC TTC GCG CTC TAC CCG GGC CCC GCG ACC TTC CTC     327
Val Glu His Asp Cys Phe Ala Leu Tyr Pro Gly Pro Ala Thr Phe Leu
        15                  20                  25

AAT GCC AGT CAG ATC TGC GAC GGA CTG CGG GGC CAC CTA ATG ACA GTG     375
Asn Ala Ser Gln Ile Cys Asp Gly Leu Arg Gly His Leu Met Thr Val
    30                  35                  40

CGC TCC TCG GTG GCT GCC GAT GTC ATT TCC TTG CTA CTG AAC GGC GAC     423
Arg Ser Ser Val Ala Ala Asp Val Ile Ser Leu Leu Leu Asn Gly Asp
45                  50                  55                  60

GGC GGC GTT GGC CGC CGG CGC CTC TGG ATC GGC CTG CAG CTG CCA CCC     471
Gly Gly Val Gly Arg Arg Arg Leu Trp Ile Gly Leu Gln Leu Pro Pro
                65                  70                  75

GGC TGC GGC GAC CCC AAG CGC CTC GGG CCC CTG CGC GGC TTC CAG TGG     519
Gly Cys Gly Asp Pro Lys Arg Leu Gly Pro Leu Arg Gly Phe Gln Trp
            80                  85                  90

GTT ACG GGA GAC AAC AAC ACC AGC TAT AGC AGG TGG GCA CGG CTC GAC     567
Val Thr Gly Asp Asn Asn Thr Ser Tyr Ser Arg Trp Ala Arg Leu Asp
        95                  100                 105

CTC AAT GGG GCT CCC CTC TGC GGC CCG TTG TGC GTC GCT GTC TCC GCT     615
Leu Asn Gly Ala Pro Leu Cys Gly Pro Leu Cys Val Ala Val Ser Ala
    110                 115                 120
```

```
GCT GAG GCC ACT GTG CCC AGC GAG CCG ATC TGG GAG GAG CAG CAG TGC      663
Ala Glu Ala Thr Val Pro Ser Glu Pro Ile Trp Glu Glu Gln Gln Cys
125                 130                 135                 140

GAA GTG AAG GCC GAT GGC TTC CTC TGC GAG TTC CAC TTC CCA GCC ACC      711
Glu Val Lys Ala Asp Gly Phe Leu Cys Glu Phe His Phe Pro Ala Thr
                145                 150                 155

TGC AGG CCA CTG GCT GTG GAG CCC GGC GCC GCG GCT GCC GCC GTC TCG      759
Cys Arg Pro Leu Ala Val Glu Pro Gly Ala Ala Ala Ala Ala Val Ser
            160                 165                 170

ATC ACC TAC GGC ACC CCG TTC GCG GCC CGC GGA GCG GAC TTC CAG GCG      807
Ile Thr Tyr Gly Thr Pro Phe Ala Ala Arg Gly Ala Asp Phe Gln Ala
        175                 180                 185

CTG CCG GTG GGC AGC TCC GCC GCG GTG GCT CCC CTC GGC TTA CAG CTA      855
Leu Pro Val Gly Ser Ser Ala Ala Val Ala Pro Leu Gly Leu Gln Leu
    190                 195                 200

ATG TGC ACC GCG CCG CCC GGA GCG GTC CAG GGG CAC TGG GCC AGG GAG      903
Met Cys Thr Ala Pro Pro Gly Ala Val Gln Gly His Trp Ala Arg Glu
205                 210                 215                 220

GCG CCG GGC GCT TGG GAC TGC AGC GTG GAG AAC GGC GGC TGC GAG CAC      951
Ala Pro Gly Ala Trp Asp Cys Ser Val Glu Asn Gly Gly Cys Glu His
                225                 230                 235

GCG TGC AAT GCG ATC CCT GGG GCT CCC CGC TGC CAG TGC CCA GCC GGC      999
Ala Cys Asn Ala Ile Pro Gly Ala Pro Arg Cys Gln Cys Pro Ala Gly
            240                 245                 250

GCC GCC CTG CAG GCA GAC GGG CGC TCC TGC ACC GCA TCC GCG ACG CAG     1047
Ala Ala Leu Gln Ala Asp Gly Arg Ser Cys Thr Ala Ser Ala Thr Gln
        255                 260                 265

TCC TGC AAC GAC CTC TGC GAG CAC TTC TGC GTT CCC AAC CCC GAC CAG     1095
Ser Cys Asn Asp Leu Cys Glu His Phe Cys Val Pro Asn Pro Asp Gln
    270                 275                 280

CCG GGC TCC TAC TCG TGC ATG TGC GAG ACC GGC TAC CGG CTG GCG GCC     1143
Pro Gly Ser Tyr Ser Cys Met Cys Glu Thr Gly Tyr Arg Leu Ala Ala
285                 290                 295                 300

GAC CAA CAC CGG TGC GAG GAC GTG GAT GAC TGC ATA CTG GAG CCC AGT     1191
Asp Gln His Arg Cys Glu Asp Val Asp Asp Cys Ile Leu Glu Pro Ser
                305                 310                 315

CCG TGT CCG CAG CGC TGT GTC AAC ACA CAG GGT GGC TTC GAG TGC CAC     1239
Pro Cys Pro Gln Arg Cys Val Asn Thr Gln Gly Gly Phe Glu Cys His
            320                 325                 330
```

```
TGC TAC CCT AAC TAC GAC CTG GTG GAC GGC GAG TGT GTG GAG CCC GTG     1287
Cys Tyr Pro Asn Tyr Asp Leu Val Asp Gly Glu Cys Val Glu Pro Val
        335                 340                 345

GAC CCG TGC TTC AGA GCC AAC TGC GAG TAC CAG TGC CAG CCC CTG AAC     1335
Asp Pro Cys Phe Arg Ala Asn Cys Glu Tyr Gln Cys Gln Pro Leu Asn
    350                 355                 360

CAA ACT AGC TAC CTC TGC GTC TGC GCC GAG GGC TTC GCG CCC ATT CCC     1383
Gln Thr Ser Tyr Leu Cys Val Cys Ala Glu Gly Phe Ala Pro Ile Pro
365                 370                 375                 380

CAC GAG CCG CAC AGG TGC CAG ATG TTT TGC AAC CAG ACT GCC TGT CCA     1431
His Glu Pro His Arg Cys Gln Met Phe Cys Asn Gln Thr Ala Cys Pro
                385                 390                 395

GCC GAC TGC GAC CCC AAC ACC CAG GCT AGC TGT GAG TGC CCT GAA GGC     1479
Ala Asp Cys Asp Pro Asn Thr Gln Ala Ser Cys Glu Cys Pro Glu Gly
            400                 405                 410

TAC ATC CTG GAC GAC GGT TTC ATC TGC ACG GAC ATC GAC GAG TGC GAA     1527
Tyr Ile Leu Asp Asp Gly Phe Ile Cys Thr Asp Ile Asp Glu Cys Glu
        415                 420                 425

AAC GGC GGC TTC TGC TCC GGG GTG TGC CAC AAC CTC CCC GGT ACC TTC     1575
Asn Gly Gly Phe Cys Ser Gly Val Cys His Asn Leu Pro Gly Thr Phe
    430                 435                 440

GAG TGC ATC TGC GGG CCC GAC TCG GCC CTT GTC CGC CAC ATT GGC ACC     1623
Glu Cys Ile Cys Gly Pro Asp Ser Ala Leu Val Arg His Ile Gly Thr
445                 450                 455                 460

GAC TGT GAC TCC GGC AAG GTC GAC GAC GAG GCC AGC GGC TCT GGC GAC     1671
Asp Cys Asp Ser Gly Lys Val Asp Asp Glu Ala Ser Gly Ser Gly Asp
                465                 470                 475

TGACTCGAG                                                           1680
```

## Claims

**Claims for the following Contracting States : AT, BE, CH, DE, DK, FR, GB, GR, IT, LI, LU, NL, SE**

1. A thrombin-binding substance having the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids and Y1 and Y2 represent any arbitrary amino acids.

**2.** The thrombin-binding substance according to Claim 1, wherein X1 is Glu, X2 is Asp, Y1 is Tyr, Y2 is Glu.

**3.** The thrombin-binding substance according to Claim 1, wherein X1 is Asp, X2 is Glu, Y1 is Ala, Y2 is Asp.

**4.** The thrombin-binding substance according to Claim 1, which is a glycosylated polypeptide.

**5.** A DNA fragment having a nucleotide sequence capable of encoding the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln

ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids and Y1 and Y2 represent any arbitrary amino acids.

6. The DNA fragment according to Claim 5, wherein X1 is Glu, X2 is Asp, Y1 is Tyr, Y2 is Glu.

7. The DNA fragment according to Claim 5, wherein X1 is Asp, X2 is Glu, Y1 is Ala, Y2 is Asp.

8. The DNA fragment having the following nucleotide sequence,

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
```

```
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780
TCCTGCACCG CATCCGCGAC GCAGTCCTGC AACGACCTCT GCGAGCACTT CTGCGTTCCC 840
AACCCCGACC AGCCGGGCTC CTACTCGTGC ATGTGCGAGA CCGGCTACCG GCTGGCGGCC 900
GACCAACACC GGTGCGAGGA CGTGGATGAC TGCATACTGG AGCCCAGTCC GTGTCCGCAG 960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG             1428
```

**9.** The DNA fragment having the following nucleotide sequence,

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
```

GGCTTACAGC TAATGTGCAC CGCGCCGCCC GGAGCGGTCC AGGGGCACTG GGCCAGGGAG 660

GCGCCGGGCG CTTGGGACTG CAGCGTGGAG AACGGCGGCT GCGAGCACGC GTGCAATGCG 720

ATCCCTGGGG CTCCCCGCTG CCAGTGCCCA GCCGGCGCCG CCCTGCAGGC AGACGGGCGC 780

TCCTGCACCG CATCCGCGAC GCAGTCCTGC AACGACCTCT GCGAGCACTT CTGCGTTCCC 840

AACCCCGACC AGCCGGGCTC CTACTCGTGC ATGTGCGAGA CCGGCTACCG GCTGGCGGCC 900

GACCAACACC GGTGCGAGGA CGTGGATGAC TGCATACTGG AGCCCAGTCC GTGTCCGCAG 960

CGCTGTGTCA ACACACAGGG TGGCTTCGAG TGCCACTGCT ACCCTAACTA CGACCTGGTG 1020

GACGGCGAGT GTGTGGAGCC CGTGGACCCG TGCTTCAGAG CCAACTGCGA GTACCAGTGC 1080

CAGCCCCTGA ACCAAACTAG CTACCTCTGC GTCTGCGCCG AGGGCTTCGC GCCCATTCCC 1140

CACGAGCCGC ACAGGTGCCA GATGTTTTGC AACCAGACTG CCTGTCCAGC CGACTGCGAC 1200

CCCAACACCC AGGCTAGCTG TGAGTGCCCT GAAGGCTACA TCCTGGACGA CGGTTTCATC 1260

TGCACGGACA TCGACGAGTG CGAAAACGGC GGCTTCTGCT CCGGGGTGTG CCACAACCTC 1320

CCCGGTACCT TCGAGTGCAT CTGCGGGCCC GACTCGGCCC TTGTCCGCCA CATTGGCACC 1380

GACTGTGACT CCGGCAAGGT CGACGACGAG GCCAGCGGCT CTGGCGAC 1428

10. A recombinant vector comprising a replicable vector and a DNA fragment having a nucleotide sequence encoding the following amino acid sequence,

AlaProAlaGluProGlnProGlyGlySerGlnCysValGluHisAspCysPheAlaLeu

TyrProGlyProAlaThrPheLeuAsnAlaSerGlnIleCysAspGlyLeuArgGlyHis

LeuMetThrValArgSerSerValAlaAlaAspValIleSerLeuLeuLeuAsnGlyAsp

GlyGlyValGlyArgArgArgLeuTrpIleGlyLeuGlnLeuProProGlyCysGlyAsp

ProLysArgLeuGlyProLeuArgGlyPheGlnTrpValThrGlyAspAsnAsnThrSer

TyrSerArgTrpAlaArgLeuAspLeuAsnGlyAlaProLeuCysGlyProLeuCysVal

AlaValSerAlaAlaGluAlaThrValProSerGluProIleTrpGluGluGlnGlnCys

GluValLysAlaAspGlyPheLeuCysGluPheHisPheProAlaThrCysArgProLeu

AlaValGluProGlyAlaAlaAlaAlaValSerIleThrTyrGlyThrProPheAla

AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids, and Y1 and Y2 represent any arbitrary amino acids.

11. A recombinant vector comprising following nucleotide sequences (1) to (7) in this order toward the downstream direction of the transcription,

(1) a nucleotide sequence acting as a promoter,
(2) a nucleotide sequence functioning as a ribosome binding site,
(3) a nucleotide sequence acting as a initiation codon,
(4) a nucleotide sequence encoding a signal peptide,
(5) a nucleotide sequence encoding the following amino acid sequence,
(6) a nucleotide sequence acting as a termination codon, and

(7) a nucleotide sequence acting as a poly A addition signal.

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu

ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids and Y1 and Y2 represent any arbitrary amino acids.

**12.** A transformed cell harboring a recombinant vector comprising a replicable vector and a DNA fragment having a nucleotide sequence encoding the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys

GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids, and Y1 and Y2 represent any arbitrary amino acids.

13. An anticoagulant composition comprising a thrombin-binding substance having the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla

AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids, and Y1 and Y2 represent any arbitrary amino acids, and a pharmaceutically acceptable carrier.

**14.** A composition for inhibiting platelet aggregation comprising a thrombin-binding substance having the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu

AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids, and Y1 and Y2 represent any arbitrary amino acids, and a pharmaceutically acceptable carrier.

**15.** A process for producing a thrombin-binding substance having the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu

AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids, and Y1 and Y2 represent any arbitrary amino acids, comprising:

cultivating a transformed cell harboring a recombinant vector comprising a replicable vector and a DNA fragment having a nucleotide sequence encoding said amino acid sequence, and collecting polypeptides produced by the cultivated cells.

**Claims for the following Contracting State : ES**

1. A process for producing a thrombin-binding substance having the following amino acid sequence,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids, and Y1 and Y2 represent any arbitrary amino acids, comprising:

cultivating a transformed cell harboring a recombinant vector comprising a replicable vector and a DNA fragment having a nucleotide sequence encoding said amino acid sequence, and
collecting polypeptides produced by the cultivated cells.

**2.** A process according to claim 1, where X1 is Glu, X2 is Asp, Y1 is Tyr; Y2 is Glu.

**3.** A process according to claim 1, where X1 is Asp, X2 is Glu, Y1 is Ala; Y2 is Asp.

**4.** A process according to claim 1, where the substance obtained is a glycosiled polypeptide.

**5.** A process according to claim 1, where the DNA fragment has the following nucleotide sequence

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
```

GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780
TCCTGCACCG CATCCGCGAC GCAGTCCTGC AACGACCTCT GCGAGCACTT CTGCGTTCCC 840
AACCCCGACC AGCCGGGCTC CTACTCGTGC ATGTGCGAGA CCGGCTACCG GCTGGCGGCC 900
GACCAACACC GGTGCGAGGA CGTGGATGAC TGCATACTGG AGCCCAGTCC GTGTCCGCAG 960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG 1428

6. A process according to claim 1, where the DNA fragment has the following nucleotide sequence

GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600

```
GGCTTACAGC TAATGTGCAC CGCGCCGCCC GGAGCGGTCC AGGGGCACTG GGCCAGGGAG  660
GCGCCGGGCG CTTGGGACTG CAGCGTGGAG AACGGCGGCT GCGAGCACGC GTGCAATGCG  720
ATCCCTGGGG CTCCCCGCTG CCAGTGCCCA GCCGGCGCCG CCCTGCAGGC AGACGGGCGC  780
TCCTGCACCG CATCCGCGAC GCAGTCCTGC AACGACCTCT GCGAGCACTT CTGCGTTCCC  840
AACCCCGACC AGCCGGGCTC CTACTCGTGC ATGTGCGAGA CCGGCTACCG GCTGGCGGCC  900
GACCAACACC GGTGCGAGGA CGTGGATGAC TGCATACTGG AGCCCAGTCC GTGTCCGCAG  960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT CGACGACGAG GCCAGCGGCT CTGGCGAC              1428
```

7. A process according to claim 1, where the recombinant vector comprises the following nucleotide sequences (1) to (7) in this order toward the downstream direction of the transcription,
   (1) a nucleotide sequence acting as a promoter,
   (2) a nucleotide sequence functioning as a ribosome binding site,
   (3) a nucleotide sequence acting as a initiation codon,
   (4) a nucleotide sequence encoding a signal peptide,
   (5) a nucleotide sequence encoding the following amino acid sequence,
   (6) a nucleotide sequence acting as a termination codon, and

(7) a nucleotide sequence acting as a poly A addition signal.

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu

ProGlyThrPheGluCysIleCysGlyProAspSerAlaLeuValArgHisIleGlyThr
AspCysAspSerGlyLysValAspX1 X2 Y1 SerGlySerGlyY2,

wherein X1 and X2 represent acidic amino acids and Y1 and Y2 represent any arbitrary amino acids.

**Patentansprüche**

**Patentansprüche für folgende Vertragsstaaten : AT, BE, CH, DE, DK, FR, GB, GR, IT, LI, LU, NL, SE**

1. Thrombin-bindende Substanz mit der folgenden Aminosäuresequenz,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle

CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen.

2. Thrombin-bindende Substanz nach Anspruch 1, in der X1 Glu ist, X2 Asp ist, Y1 Tyr ist, Y2 Glu ist.

3. Thrombin-bindende Substanz nach Anspruch 1, in der X1 Asp ist, X2 Glu ist, Y1 Ala ist, Y2 Asp ist.

4. Thrombin-bindende Substanz nach Anspruch 1, die ein glykosyliertes Polypeptid ist.

5. DNA-Fragment mit einer Nukleotid-Sequenz, die in der Lage ist, für die folgende Aminosäuresequenz zu codieren,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg

SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen.

**6.** DNA-Fragment nach Anspruch 5, worin X1 Glu ist, X2 Asp ist, Y1 Tyr ist, Y2 Glu ist.

**7.** DNA-Fragment nach Anspruch 5, worin X1 Asp ist, X2 Glu ist, Y1 Ala ist, Y2 Asp ist.

**8.** DNA-Fragment mit der folgenden Nukleotidsequenz

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
```

GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780
TCCTGCACCG CATCCGCGAC GCAGTCCTGC AACGACCTCT GCGAGCACTT CTGCGTTCCC 840
AACCCCGACC AGCCGGGCTC CTACTCGTGC ATGTGCGAGA CCGGCTACCG GCTGGCGGCC 900
GACCAACACC GGTGCGAGGA CGTGGATGAC TGCATACTGG AGCCCAGTCC GTGTCCGCAG 960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG 1428

**9.** DNA-Fragment mit der folgenden Nukleotidsequenz

GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600

```
GGCTTACAGC TAATGTGCAC CGCGCCGCCC GGAGCGGTCC AGGGGCACTG GGCCAGGGAG  660
GCGCCGGGCG CTTGGGACTG CAGCGTGGAG AACGGCGGCT GCGAGCACGC GTGCAATGCG  720
ATCCCTGGGG CTCCCCGCTG CCAGTGCCCA GCCGGCGCCG CCCTGCAGGC AGACGGGCGC  780
TCCTGCACCG CATCCGCGAC GCAGTCCTGC AACGACCTCT GCGAGCACTT CTGCGTTCCC  840
AACCCCGACC AGCCGGGCTC CTACTCGTGC ATGTGCGAGA CCGGCTACCG GCTGGCGGCC  900
GACCAACACC GGTGCGAGGA CGTGGATGAC TGCATACTGG AGCCCAGTCC GTGTCCGCAG  960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT CGACGACGAG GCCAGCGGCT CTGGCGAC             1428
```

**10.** Rekombinanter Vektor, umfassend einen replizierbaren Vektor und ein DNA-Fragment mit einer Nukleotidsequenz, die für die folgende Aminosäuresequenz codiert,

```
AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
```

AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen.

**11.** Rekombinanter Vektor, der die folgenden Nukleotidsequenzen (1) bis (7) in dieser Reihenfolge gegen die Stromabwärts-Richtung der Transkription hin umfaßt,

(1) eine Nukleotidsequenz, die als Promotor wirkt,
(2) eine Nukleotidsequenz, die die Funktion einer Ribosomen-Bindungsstelle aufweist,
(3) eine Nukleotidsequenz, die als Initiationscodon wirkt,
(4) eine Nukleotidsequenz, die für ein Signalpeptid codiert,
(5) eine Nukleotidsequenz, die für die folgende Aminosäuresequenz codiert,
(6) eine Nukleotidsequenz, die als Terminator-Codon wirkt, und

(7) eine Nukleotidsequenz, die als Poly-A-Additionssignal wirkt,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen.

**12.** Transformierte Zelle, die einen rekombinanten Vektor beherbergt, der einen replizierbaren Vektor und ein DNA-Fragment mit einer Nukleotidsequenz umfaßt, die für die folgende Aminosäuresequenz codiert,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle

CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen.

**13.** Antikoagulans-Zusammensetzung, die eine Thrombin-bindende Substanz mit der folgenden Aminosäuresequenz

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys

GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen, und einen pharmazeutisch annehmbaren Träger umfaßt.

**14.** Zusammensetzung zur Hemmung der Blutplättchen-Aggregation, die eine Thrombin-bindende Substanz mit der folgenden Aminosäuresequenz,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla

AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen, und einen pharmazeutisch annehmbaren Träger umfaßt.

**15.** Verfahren zur Herstellung einer Thrombin-bindenden Substanz mit der folgenden Aminosäuresequenz,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla

IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen, umfassend:

das Kultivieren einer transformierten Zelle, die einen rekombinanten Vektor beherbergt, der einen replizierbaren Vektor und ein DNA-Fragment mit einer Nukleotidsequenz umfaßt, die für besagte Aminosäuresequenz codiert, und

das Sammeln von Polypeptiden, die von den kultivierten Zellen erzeugt wurden.

**Patentansprüche für folgenden Vertragsstaat: ES**

1. Verfahren zur Herstellung einer Thrombin-bindenden Substanz mit der folgenden Aminosäuresequenz,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen, umfassend:

das Kultivieren einer transformierten Zelle, die einen rekombinanten Vektor beherbergt, der einen replizierbaren Vektor und ein DNA-Fragment mit einer Nukleotidsequenz umfaßt, die für besagte Aminosäuresequenz codiert, und

das Sammeln von Polypeptiden, die von den kultivierten Zellen erzeugt wurden.

**2.** Verfahren nach Anspruch 1, in dem X1 Glu ist, X2 Asp ist, Y1 Tyr ist, Y2 Glu ist.

**3.** Verfahren nach Anspruch 1, in dem X1 Asp ist, X2 Glu ist, Y1 Ala ist, Y2 Asp ist.

**4.** Verfahren nach Anspruch 1, in dem die erhaltene Substanz ein glykosyliertes Polypeptid ist.

**5.** Verfahren nach Anspruch 1, in dem das DNA-Fragment die folgende Nukleotidsequenz aufweist

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
```

```
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780
TCCTGCACCG CATCCGCGAC GCAGTCCTGC AACGACCTCT GCGAGCACTT CTGCGTTCCC 840
AACCCCGACC AGCCGGGCTC CTACTCGTGC ATGTGCGAGA CCGGCTACCG GCTGGCGGCC 900
GACCAACACC GGTGCGAGGA CGTGGATGAC TGCATACTGG AGCCCAGTCC GTGTCCGCAG 960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG             1428
```

**6.** Verfahren nach Anspruch 1, in dem das DNA-Fragment die folgende Nukleotidsequenz aufweist

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
```

```
GGCTTACAGC TAATGTGCAC CGCGCCGCCC GGAGCGGTCC AGGGGCACTG GGCCAGGGAG  660
GCGCCGGGCG CTTGGGACTG CAGCGTGGAG AACGGCGGCT GCGAGCACGC GTGCAATGCG  720
ATCCCTGGGG CTCCCCGCTG CCAGTGCCCA GCCGGCGCCG CCCTGCAGGC AGACGGGCGC  780
TCCTGCACCG CATCCGCGAC GCAGTCCTGC AACGACCTCT GCGAGCACTT CTGCGTTCCC  840
AACCCCGACC AGCCGGGCTC CTACTCGTGC ATGTGCGAGA CCGGCTACCG GCTGGCGGCC  900
GACCAACACC GGTGCGAGGA CGTGGATGAC TGCATACTGG AGCCCAGTCC GTGTCCGCAG  960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT CGACGACGAG GCCAGCGGCT CTGGCGAC              1428
```

**7.** Verfahren nach Anspruch 1, in dem der rekombinante Vektor die folgenden Nukleotidsequenzen (1) bis (7) in dieser Reihenfolge gegen die Stromabwärts-Richtung der Transkription hin umfaßt,

(1) eine Nukleotidsequenz, die als Promotor wirkt,
(2) eine Nukleotidsequenz, die die Funktion einer Ribosomen-Bindungsstelle aufweist,
(3) eine Nukleotidsequenz, die als Initiationscodon wirkt,
(4) eine Nukleotidsequenz, die für ein Signalpeptid codiert,
(5) eine Nukleotidsequenz, die für die folgende Aminosäuresequenz codiert,
(6) eine Nukleotidsequenz, die als Terminator-Codon wirkt, und

(7) eine Nukleotidsequenz, die als Poly-A-Additionssignal wirkt,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

in der X1 und X2 saure Aminosäuren darstellen und Y1 und Y2 irgendwelche beliebigen Aminosäuren darstellen.

**Revendications**

**Revendications pour les Etats contractants suivants : AT, BE, CH, DE, DK, FR, GB, GR, IT, LI, LU, NL, SE**

**1.** Substance liant la thrombine, ayant la séquence suivante d'acides aminés,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides et Y1 et Y2 représentent des acides aminés quelconques.

2. Substance liant la thrombine selon la revendication 1, dans laquelle X1 est Glu, X2 est Asp, Y1 est Tyr, Y2 est Glu.

3. Substance liant la thrombine selon la revendication 1, dans laquelle X1 est Asp, X2 est Glu, Y1 est Ala, Y2 est Asp.

4. Substance liant la thrombine selon la revendication 1, qui est un polypeptide glycosylé.

5. Fragment d'ADN ayant une séquence de nucléotides capable de coder pour la séquence suivante d'acides aminés

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal

AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides et Y1 et Y2 représentent des acides aminés quelconques.

6. Fragment d'ADN selon la revendication 5, dans lequel X1 est Glu, X2 est Asp, Y1 est Tyr, Y2 est Glu.

7. Fragment d'ADN selon la revendication 5, dans lequel X1 est Asp, X2 est Glu, Y1 est Ala, Y2 est Asp.

8. Fragment d'ADN ayant la séquence de nucléotides suivante,

GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
GGCTTACAGC TAATGTGCAC CGCGCCGCCC GGAGCGGTCC AGGGGCACTG GCCAGGGAG 660
GCGCCGGGCG CTTGGGACTG CAGCGTGGAG AACGGCGGCT GCGAGCACGC GTGCAATGCG 720

ATCCCTGGGG CTCCCCGCTG CCAGTGCCCA GCCGGCGCCG CCCTGCAGGC AGACGGGCGC 780
TCCTGCACCG CATCCGCGAC GCAGTCCTGC AACGACCTCT GCGAGCACTT CTGCGTTCCC 840
AACCCCGACC AGCCGGGCTC CTACTCGTGC ATGTGCGAGA CCGGCTACCG GCTGGCGGCC 900
GACCAACACC GGTGCGAGGA CGTGGATGAC TGCATACTGG AGCCCAGTCC GTGTCCGCAG 960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG 1428

**9.** Fragment d'ADN ayant la séquence de nucléotides suivante,

GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720

ATCCCTGGGG CTCCCCGCTG CCAGTGCCCA GCCGGCGCCG CCCTGCAGGC AGACGGGCGC 780

TCCTGCACCG CATCCGCGAC GCAGTCCTGC AACGACCTCT GCGAGCACTT CTGCGTTCCC 840

AACCCCGACC AGCCGGGCTC CTACTCGTGC ATGTGCGAGA CCGGCTACCG GCTGGCGGCC 900

GACCAACACC GGTGCGAGGA CGTGGATGAC TGCATACTGG AGCCCAGTCC GTGTCCGCAG 960

CGCTGTGTCA ACACACAGGG TGGCTTCGAG TGCCACTGCT ACCCTAACTA CGACCTGGTG 1020

GACGGCGAGT GTGTGGAGCC CGTGGACCCG TGCTTCAGAG CCAACTGCGA GTACCAGTGC 1080

CAGCCCCTGA ACCAAACTAG CTACCTCTGC GTCTGCGCCG AGGGCTTCGC GCCCATTCCC 1140

CACGAGCCGC ACAGGTGCCA GATGTTTTGC AACCAGACTG CCTGTCCAGC CGACTGCGAC 1200

CCCAACACCC AGGCTAGCTG TGAGTGCCCT GAAGGCTACA TCCTGGACGA CGGTTTCATC 1260

TGCACGGACA TCGACGAGTG CGAAAACGGC GGCTTCTGCT CCGGGGTGTG CCACAACCTC 1320

CCCGGTACCT TCGAGTGCAT CTGCGGGCCC GACTCGGCCC TTGTCCGCCA CATTGGCACC 1380

GACTGTGACT CCGGCAAGGT CGACGACGAG GCCAGCGGCT CTGGCGAC 1428

**10.** Vecteur recombinant comprenant un vecteur réplicable et un fragment d'ADN ayant une séquence de nucléotides codant pour la séquence suivante d'acides aminés,

AlaProAlaGluProGlnProGlyGlySerGlnCysValGluHisAspCysPheAlaLeu

TyrProGlyProAlaThrPheLeuAsnAlaSerGlnIleCysAspGlyLeuArgGlyHis

LeuMetThrValArgSerSerValAlaAlaAspValIleSerLeuLeuLeuAsnGlyAsp

GlyGlyValGlyArgArgArgLeuTrpIleGlyLeuGlnLeuProProGlyCysGlyAsp

ProLysArgLeuGlyProLeuArgGlyPheGlnTrpValThrGlyAspAsnAsnThrSer

TyrSerArgTrpAlaArgLeuAspLeuAsnGlyAlaProLeuCysGlyProLeuCysVal

AlaValSerAlaAlaGluAlaThrValProSerGluProIleTrpGluGluGlnGlnCys

GluValLysAlaAspGlyPheLeuCysGluPheHisPheProAlaThrCysArgProLeu

AlaValGluProGlyAlaAlaAlaAlaValSerIleThrTyrGlyThrProPheAla

AlaArgGlyAlaAspPheGlnAlaLeuProValGlySerSerAlaAlaValAlaProLeu

GlyLeuGlnLeuMetCysThrAlaProProGlyAlaValGlnGlyHisTrpAlaArgGlu

AlaProGlyAlaTrpAspCysSerValGluAsnGlyGlyCysGluHisAlaCysAsnAla

IleProGlyAlaProArgCysGlnCysProAlaGlyAlaAlaLeuGlnAlaAspGlyArg

SerCysThrAlaSerAlaThrGlnSerCysAsnAspLeuCysGluHisPheCysValPro

AsnProAspGlnProGlySerTyrSerCysMetCysGluThrGlyTyrArgLeuAlaAla

AspGlnHisArgCysGluAspValAspAspCysIleLeuGluProSerProCysProGln

ArgCysValAsnThrGlnGlyGlyPheGluCysHisCysTyrProAsnTyrAspLeuVal

AspGlyGluCysValGluProValAspProCysPheArgAlaAsnCysGluTyrGlnCys

GlnProLeuAsnGlnThrSerTyrLeuCysValCysAlaGluGlyPheAlaProIlePro

HisGluProHisArgCysGlnMetPheCysAsnGlnThrAlaCysProAlaAspCysAsp

ProAsnThrGlnAlaSerCysGluCysProGluGlyTyrIleLeuAspAspGlyPheIle

CysThrAspIleAspGluCysGluAsnGlyGlyPheCysSerGlyValCysHisAsnLeu

ProGlyThrPheGluCysIleCysGlyProAspSerAlaLeuValArgHisIleGlyThr

AspCysAspSerGlyLysValAspX1 X2 Y1 SerGlySerGlyY2,

dans laquelle X1 et X2 représentent des acides aminés acides et Y1 et Y2 représentent des acides aminés quelconques.

**11.** Vecteur recombinant comprenant les séquences suivantes de nucléotides (1) à (7) dans cet ordre en direction vers l'aval de la transcription,

(1) une séquence de nucléotides agissant comme promoteur,
(2) une séquence de nucléotides agissant comme site de liaison du ribosome,
(3) une séquence de nucléotides agissant comme codon d'initiation,
(4) une séquence de nucléotides codant pour un peptide signal,
(5) une séquence de nucléotides codant pour la séquence d'acides aminés suivante,
(6) une séquence de nucléotides agissant comme codon d'arrêt, et

(7) une séquence de nucléotides agissant comme signal d'addition poly A

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides et Y1 et Y2 représentent des acides aminés quelconques.

**12.** Cellule transformée contenant un vecteur recombinant comprenant un vecteur réplicable et un fragment d'ADN ayant une séquence de nucléotides codant pour la séquence suivante d'acides aminés,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides, et Y1 et Y2 représentent des acides aminés quelconques.

**13.** Composition anticoagulante comprenant une substance liant la thrombine, ayant la séquence suivante d'acides aminés,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu

ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides, et Y1 et Y2 représentent des acides aminés quelconques et un support pharmaceutiquement acceptable.

**14.** Composition pour inhiber l'agrégation des plaquettes comprenant une substance liant la thrombine ayant la séquence suivante d'acides aminés,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro

HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides, et Y1 et Y2 représentent des acides aminés quelconques, et un support pharmaceutiquement acceptable.

**15.** Procédé de production d'une substance liant la thrombine, ayant la séquence suivante d'acides aminés,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro

HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides, et Y1 et Y2 représentent des acides aminés quelconques, comprenant les opérations suivantes:

cultiver une cellule transformée contenant un vecteur recombinant comprenant un vecteur réplicable et un fragment d'ADN ayant une séquence de nucléotides codant pour ladite séquence d'acides aminés; et

recueillir les polypeptides produits par les cellules cultivées.

**Revendications pour l'Etat contractant suivant : ES**

1. Procédé de production d'une substance liant la thrombine, ayant la séquence suivante d'acides aminés,

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu

AlaValGluProGlyAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés acides, et Y1 et Y2 représentent des acides aminés quelconques, comprenant les opérations suivantes:

cultiver une cellule transformée contenant un vecteur recombinant comprenant un vecteur réplicable et un fragment d'ADN ayant une séquence de nucléotides codant pour ladite séquence d'acides aminés; et

recueillir les polypeptides produits par les cellules cultivées.

2. Procédé selon la revendication 1, dans lequel X1 est Glu, X2 est Asp, Y1 est Tyr, Y2 est Glu.

3. Procédé selon la revendication 1, dans lequel X1 est Asp, X2 est Glu, Y1 est Ala, Y2 est Asp.

4. Procédé selon la revendication 1, dans lequel la substance obtenue est un polypeptide glycosylé.

5. Procédé selon la revendication 1, dans lequel le fragment d'ADN a la séquence de nucléotides suivante

```
GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780
TCCTGCACCG CATCCGCGAC GCAGTCCTGC AACGACCTCT GCGAGCACTT CTGCGTTCCC 840
```

AACCCCGACC AGCCGGGCTC CTACTCGTGC ATGTGCGAGA CCGGCTACCG GCTGGCGGCC 900
GACCAACACC GGTGCGAGGA CGTGGATGAC TGCATACTGG AGCCCAGTCC GTGTCCGCAG 960
CGCTGTGTCA ACACACAGGG TGGCTTCGAG TGCCACTGCT ACCCTAACTA CGACCTGGTG 1020
GACGGCGAGT GTGTGGAGCC CGTGGACCCG TGCTTCAGAG CCAACTGCGA GTACCAGTGC 1080
CAGCCCCTGA ACCAAACTAG CTACCTCTGC GTCTGCGCCG AGGGCTTCGC GCCCATTCCC 1140
CACGAGCCGC ACAGGTGCCA GATGTTTTGC AACCAGACTG CCTGTCCAGC CGACTGCGAC 1200
CCCAACACCC AGGCTAGCTG TGAGTGCCCT GAAGGCTACA TCCTGGACGA CGGTTTCATC 1260
TGCACGGACA TCGACGAGTG CGAAAACGGC GGCTTCTGCT CCGGGGTGTG CCACAACCTC 1320
CCCGGTACCT TCGAGTGCAT CTGCGGGCCC GACTCGGCCC TTGTCCGCCA CATTGGCACC 1380
GACTGTGACT CCGGCAAGGT GGACGAGGAC TATAGCGGCT CTGGCGAG 1428

**6.** Procédé selon la revendication 1, dans lequel le fragment d'ADN a la séquence de nucléotides suivante

GCACCCGCAG AGCCGCAGCC GGGTGGCAGC CAGTGCGTCG AGCACGACTG CTTCGCGCTC 60
TACCCGGGCC CCGCGACCTT CCTCAATGCC AGTCAGATCT GCGACGGACT GCGGGGCCAC 120
CTAATGACAG TGCGCTCCTC GGTGGCTGCC GATGTCATTT CCTTGCTACT GAACGGCGAC 180
GGCGGCGTTG GCCGCCGGCG CCTCTGGATC GGCCTGCAGC TGCCACCCGG CTGCGGCGAC 240
CCCAAGCGCC TCGGGCCCCT GCGCGGCTTC CAGTGGGTTA CGGGAGACAA CAACACCAGC 300
TATAGCAGGT GGGCACGGCT CGACCTCAAT GGGGCTCCCC TCTGCGGCCC GTTGTGCGTC 360
GCTGTCTCCG CTGCTGAGGC CACTGTGCCC AGCGAGCCGA TCTGGGAGGA GCAGCAGTGC 420
GAAGTGAAGG CCGATGGCTT CCTCTGCGAG TTCCACTTCC CAGCCACCTG CAGGCCACTG 480
GCTGTGGAGC CCGGCGCCGC GGCTGCCGCC GTCTCGATCA CCTACGGCAC CCCGTTCGCG 540
GCCCGCGGAG CGGACTTCCA GGCGCTGCCG GTGGGCAGCT CCGCCGCGGT GGCTCCCCTC 600
GGCTTACAGC TAATGTGCAC CGCGCCGCCC GGAGCGGTCC AGGGGCACTG GGCCAGGGAG 660
GCGCCGGGCG CTTGGGACTG CAGCGTGGAG AACGGCGGCT GCGAGCACGC GTGCAATGCG 720
ATCCCTGGGG CTCCCCGCTG CCAGTGCCCA GCCGGCGCCG CCCTGCAGGC AGACGGGCGC 780

TCCTGCACCG CATCCGCGAC GCAGTCCTGC AACGACCTCT GCGAGCACTT CTGCGTTCCC 840

AACCCCGACC AGCCGGGCTC CTACTCGTGC ATGTGCGAGA CCGGCTACCG GCTGGCGGCC 900

GACCAACACC GGTGCGAGGA CGTGGATGAC TGCATACTGG AGCCCAGTCC GTGTCCGCAG 960

CGCTGTGTCA ACACACAGGG TGGCTTCGAG TGCCACTGCT ACCCTAACTA CGACCTGGTG 1020

GACGGCGAGT GTGTGGAGCC CGTGGACCCG TGCTTCAGAG CCAACTGCGA GTACCAGTGC 1080

CAGCCCCTGA ACCAAACTAG CTACCTCTGC GTCTGCGCCG AGGGCTTCGC GCCCATTCCC 1140

CACGAGCCGC ACAGGTGCCA GATGTTTTGC AACCAGACTG CCTGTCCAGC CGACTGCGAC 1200

CCCAACACCC AGGCTAGCTG TGAGTGCCCT GAAGGCTACA TCCTGGACGA CGGTTTCATC 1260

TGCACGGACA TCGACGAGTG CGAAAACGGC GGCTTCTGCT CCGGGGTGTG CCACAACCTC 1320

CCCGGTACCT TCGAGTGCAT CTGCGGGCCC GACTCGGCCC TTGTCCGCCA CATTGGCACC 1380

GACTGTGACT CCGGCAAGGT CGACGACGAG GCCAGCGGCT CTGGCGAC 1428

**7.** Procédé selon la revendication 1 dans lequel le vecteur recombinant comprend les séquences suivantes de nucléotides (1) à (7) dans cet ordre vers la direction aval de la transcription,

(1) une séquence de nucléotides agissant comme promoteur,
(2) une séquence de nucléotides agissant comme site de liaison du ribosome,
(3) une séquence de nucléotides agissant comme codon d'initiation,
(4) une séquence de nucléotides codant pour un peptide signal,
(5) une séquence de nucléotides codant pour la séquence d'acides aminés suivante,
(6) une séquence de nucléotides agissant comme codon d'arrêt, et
(7) une séquence de nucléotides agissant comme signal d'addition de poly A

AlaProAlaGluProGlnProGlyGlySerGlnCysValGluHisAspCysPheAlaLeu

TyrProGlyProAlaThrPheLeuAsnAlaSerGlnIleCysAspGlyLeuArgGlyHis

LeuMetThrValArgSerSerValAlaAlaAspValIleSerLeuLeuLeuAsnGlyAsp

GlyGlyValGlyArgArgArgLeuTrpIleGlyLeuGlnLeuProProGlyCysGlyAsp

ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAspX1 X2 Y1 SerGlySerGlyY2,

dans laquelle X1 et X2 représentent des acides aminés et Y1 et Y2 représentent des acides aminés quelconques.

Fig. 1

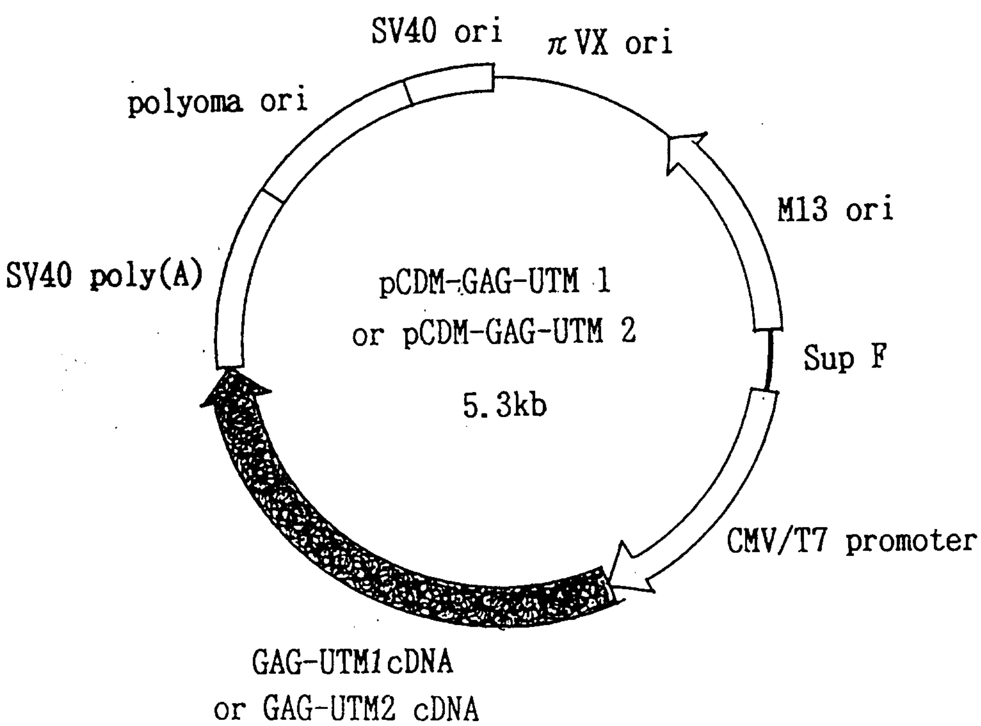
SV40 ori
πVX ori
polyoma ori
M13 ori
SV40 poly(A)
pCDM-GAG-UTM 1
or pCDM-GAG-UTM 2
5.3kb
Sup F
CMV/T7 promoter
GAG-UTM1 cDNA
or GAG-UTM2 cDNA